# EUROPEAN PATENT SPECIFICATION

(11) **EP 1 709 170 B1**
(45) Date of publication and mention of the grant of the patent: **21.02.2018**
(21) Application number: 05705852.1
(22) Date of filing: 18.01.2005
(51) Int. Cl.: C12N 15/10, C12N 15/63, C12N 15/64, C12N 15/67, C12N 15/74, C12N 1/21

(54) **EXPRESSION OF MAMMALIAN PROTEINS IN PSEUDOMONAS FLUORESCENS**
EXPRESSION VON SÄUGERPROTEINEN IN PSEUDOMONAS FLUORESCENS
EXPRESSION DE PROTEINES MAMMIFERES DANS PSEUDOMONAS FLUORESCENS

(30) Priority: 16.01.2004 US 537148 P; 22.04.2004 US 564798 P
(43) Date of publication of application: 11.10.2006
(60) Divisional application: 11176612.7
(73) Proprietor: Pfenex Inc., San Diego, CA 92121 (US)
(72) Inventor: RETALLACK, Diane, M., Poway, CA 92064 (US); SQUIRES, Charles, H., Poway, CA 92064 (US); WATKINS, David, C., East Greenwich, RI 02818 (US); GAERTNER, Frank, H., San Diego, CA 92109 (US); LEE, Stacey, Lynn, San Diego, CA 92129 (US); SHUTTER, Robert, Poway, CA 92064 (US)
(74) Representative: HGF Limited
(86) International application number: PCT/US2005/001549
(87) International publication number: WO 2005/069913

(56) References cited:
- US-A- 5 232 840
- US-A- 5 441 934
- US-A- 5 527 883
- CASAVANT N CAROL ET AL: "Use of a site-specific recombination-based biosensor for detecting bioavailable toluene and related compounds on roots." ENVIRONMENTAL MICROBIOLOGY APR 2003, vol. 5, no. 4, April 2003 (2003-04), pages 238-249, XP002473227 ISSN: 1462-2912
- NAKASHIMA N ET AL: "Cell-free protein synthesis using cell extract of Pseudomonas fluorescens and CspA promoter" BIOCHEMICAL AND BIOPHYSICAL RESEARCH COMMUNICATIONS, ACADEMIC PRESS INC. ORLANDO, FL, US, vol. 319, no. 2, 25 June 2004 (2004-06-25), pages 671-676, XP004512488 ISSN: 0006-291X
- GAERTNER ET AL: "Amended recombinant cells (ARCs(TM)): An economical and surprisingly effective production and delivery vehicle for recombinant bovine IFN-gamma" JOURNAL OF CONTROLLED RELEASE, ELSEVIER, AMSTERDAM, NL, vol. 107, no. 2, 3 October 2005 (2005-10-03), pages 189-202, XP005076213 ISSN: 0168-3659
- RETALLACK DIANE ET AL: "Pseudomonas fluorescens - a robust expression platform for pharmaceutical protein production" MICROBIAL CELL FACTORIES, BIOMED CENTRAL, LONDON, NL, vol. 5, no. Suppl 1, 10 October 2006 (2006-10-10), page S28, XP021024053 ISSN: 1475-2859
- RETALLACK DIANE M ET AL: "Transport of heterologous proteins to the periplasmic space of Pseudomonas fluorescens using a variety of native signal sequences" BIOTECHNOLOGY LETTERS, vol. 29, no. 10, October 2007 (2007-10), pages 1483-1491, XP002473228 ISSN: 0141-5492
- AHN J.H. ET AL: 'Homologous Expression of the Lipase and ABC Transporter Gene Cluster, tliDEFA, Enhances Lipase Secretion in Pseudomonas.' APPLIED AND ENVIRONMENTAL MICROBIOLOGY. vol. 67, no. 12, December 2001, pages 5506 - 5511, XP002988919

## Description

### REFERENCE TO PRIOR APPLICATIONS

This application claims priority to U.S. Provisional Application Nos. 60/564,798, entitled "Expression of Mammalian Proteins in *Pseudomonas fluorescens,* " filed April 22, 2004, and 60/537,148, entitled "Protein Expression Systems," filed January 16, 2004.

### FIELD OF THE INVENTION

The invention is a process for improved production of a recombinant mammalian protein by expression in a *Pseudomonas fluorescens* organism. The process improves production of mammalian protein expression over known expression systems.
The invention also relates to a *Pseudomonas fluorescens* cell comprising a nucleic acid encoding a recombinant human peptide.

### BACKGROUND OF THE INVENTION

More than 325 million people worldwide have been helped by the more than 155 biotechnology drugs and vaccines approved by the U.S. Food and Drug Administration (FDA). In addition, there are more than 370 biotech drug products and vaccines currently in clinical trials targeting more than 200 diseases, including various cancers, Alzheimer's disease, heart disease, diabetes, multiple sclerosis, AIDS and arthritis. Unlike traditional small molecule therapeutics that are produced through classical chemical synthesis, proteins are usually produced in living cells inefficiently and at high cost. Due to the high cost and complexity, there is a shortage of manufacturing capacity for protein-based therapeutics.

The use of microbial cells to produce products has a very long history. As early as 1897, Buchner discovered that enzymes extracted from yeast are effective in converting sugar into alcohol, leading to the production of key industrial chemicals using microorganisms. By the 1940s, large-scale production of penicillin via fermentation was achieved. Techniques for the insertion of foreign genes into bacteria were first developed in the early 1970s. Bacterial production of commercially viable recombinant mammalian protein was first exploited in the production of human insulin (Goeddel, et al., 1979a; Wong, 1997). Today fermentation and cell culture underlie the bulk of the industry's production of alcohol, antibiotics, biochemicals and therapeutic proteins. However, development and manufacturing of therapeutically useful proteins has been hampered due, in large part, to the limitations of the current organisms used to express these exogenous proteins.

### Prokaryotic vs. Eukaryotic Protein Expression

Although bacterial expression system are often used to produce recombinant eukaryotic proteins, typically the proteins yielded differ significantly from their original counterparts. In general, it is a challenge to reproduce the eukaryotic secondary and tertiary structures in *E. coli* expression systems. At the same time, while the eukaryotic expression systems currently are better able to form the secondary and tertiary structures of recombinant eukaryotic proteins, the capacity of these systems to produce recombinant proteins in large quantity is limited.

Post-translational modifications represent the most significant differences between prokaryotic and eukaryotic protein expression. Prokaryotes (i.e., bacteria) have a very simply cellular structure and no membrane-bound organelles. In eukaryotes, a protein is often modified after it is intially produced. These modifications, in many cases, are necessary to convert the peptide into a functional form. Thus, even when exisiting bacterial expression systems produce a protein with the correct primary structure, the protein may not be post-translationally modified and is therefore often nonfunctional. Common modifications include disulfide bond formation, glycosylation, acetylation, acylation, phosphorylation, and gamma-carboxylation, all of which can regulate protein folding and biological activity. Bacterial expression systems generally do not properly glycosylate, acetylate, acylate, phosphorylate, or gamma-carboxylate eukaryotic proteins.

Bacteria, such as *E. coli,* can form disulfide bonds, but the bonds are often formed in the incorrect configuration required for biological activity; therefore, denaturation and refolding is usually required to produce active eukaryotic proteins. Molecular chaperone proteins are present in both prokaryotes and eukaryotes that facilitate the folding of other proteins. In the absence of such chaperones, unfolded or partially folded polypeptide chains are unstable within the cell, frequently folding incorrectly or aggregating into insoluble complexes. The binding of chaperones stabilizes these unfolded polypeptides, thereby preventing incorrect folding or aggregation and allowing the polypeptide chain to fold into its correct conformation. However, chaperones differ in each type of cell, and can be differentially expressed based on extracellular conditions.

### Problems With Current Expression Systems

*Escherichia coli* (*E. coli*) is the most widely and routinely used protein expression system. Production in *E. coli* is inexpensive, fast, and well characterized. Further, scale-up and harvesting is possible and cGMP production is well established. However, there are significant limitations to the use of *E. coli,* which often prove difficult to overcome, particularly when expressing recombinant mammalian proteins.

Along with the limitations described above, the high-level expression of recombinant gene products in *E. coli* often results in the misfolding of the protein of interest and its subsequent degradation by cellular proteases or deposition into biologically inactive aggregates known as inclusion bodies. Protein found in inclusion bodies typically must be extracted and renautred for activity, adding time and expense to the process. Typical renaturation methods involve attempts to dissolve the aggregate in concentrated denaturant, and subsequent removal of the denaturant by dilution. Some of the factors which have been suggested to be involved in inclusion body formation include the high local concentration of protein; a reducing environment in the cytoplasm (*E. coli* cytoplasm has a high level of glutathione) preventing formation of disulfide bonds; lack of post-translational modifications, which can increase the protein solubility; improper interactions with chaperones and other enzymes involved in *in vivo* folding; intermolecular cross-linking via disulfide or other covalent bonds; and increased aggregation of folding intermediates due to their limited solubility. It is probably a combination of these factors, as well as a limited availability of chaperones, which most commonly lead to the formation of inclusion bodies.

Yeast expression systems, such as *Saccharomyces cerevisiae* or *Pichia pastoris,* are also commonly used to produce proteins. These systems are well characterized, provide good expression levels and are relatively fast and inexpensive compared to other eukaryotic ecpression systems. However, yeast can accomplish only limited post-translational protein modifications, the protein may need refolding, and harvesting of the protein can be a problem due to the characteristics of the cell wall.

Insect cell expression systems have also emerged as an attractive, but expensive, alternative as a protein expression system. Correctly folded proteins that are generally post-translationally modified can sometimes be produced and extracellular expression has been achieved. However, it is not as rapid as bacteria and yeast, and scale-up is generally challenging.

Mammalian cell expression systems, such as Chinese hamster ovary cells, are often used for complex protein expression. This system usually produces correctly folded proteins with the appropriate post-translational modifications and the proteins can be expressed extracellularly. However, the system is very expensive, scale-up is slow and often not feasible, and protein yields are lower than in any other system.

### Pseudomonas fluorescens (P. fluorescens)

*Pseudomonas fluorescens* encompasses a group of common, nonpathogenic saprophytes that colonize soil, water and plant surface environments. *P. fluorescens* are extensively used in agricultural and industrial processes, including commercially for the production of non-mammalian industrial and agricultural proteins. Nonmammalian enzymes derived from *P. fluorescens* have been used to reduce environmental contamination, as detergent additives, and for stereoselective hydrolysis. Mycogen began expressing recombinant bacterial proteins in *P. fluorescens* in the mid-1980's and filed its first patent application on the expression of the *Bacillus thuringiensis* toxin in *P. fluorescens* on January 22, 1985 ("Cellular encapsulation of biological pesticides"). Between 1985 and 2004, Mycogen, later Dow Agro Sciences, as well as other companies, capitalized on the agricultural use of *P. fluorescens* in patent applications on the production of pesticidal, insecticidal, and nematocidal toxins, as well as on specific toxic sequences and genetic manipulation to enhance expression of these. Examples of patent applications directed to the expression of recombinant bacterial proteins in *P. fluorescens* include: U.S. Patent Nos. 3,844,893; 3,878,093, 4,169,010; 5,292,507; 5,558,862; 5,559,015; 5,610,044; 5,622,846; 5,643,774; 5,662,898; 5,677,127; 5,686,282; 3,844,893; 3,878,093; 4,169,010; 5,232,840; 5,292,507; 5,558,862; 5,559,015; 5,610,044; 5,622,846; 5,643,774; 5,662,898; 5,677,127; 5,686,282; 5,686,283; 5,698,425; 5,710,031; 5,728,574; 5,731,280; 5,741,663; 5,756,087; 5,766,926; 5,824,472; 5,869,038; 5,891,688; 5,952,208; 5,955,348; 6,051,383; 6,117,670; 6,184,440; 6,194,194; 6,268,549; 6,277,625; 6,329,172; 6,447,770; as well as PCT Publication Nos. WO 00/15761; WO 00/29604; WO 01/27258; WO 02/068660; WO 02/14551; WO 02/16940; WO 03/089455; WO 04/006657; WO 04/011628; WO 87/05937; WO 87/05938; WO 95/03395; WO 98/24919; WO 99/09834; and WO 99/53035.

On October 8, 2003, Dow AgroSciences filed PCT Publication No. 04/087864 entitled, "Amended Recombinant Cells (ARCs) for the Production and Delivery of Antiviral Agents, Adjuvants and Vaccine Accelerants". The application describes recombinant cells that can include at least one heterologous gene encoding a chemokine or a cytokine and the administration of such cells to a host to accelerate an immune response. The application demonstrates the production of bovine interferon-a and interferon-γ in *P. fluorescens.*

Dow Global Technologies currently has several pending patent applications in the area of use of *P. fluorescens* to produce recombinant proteins. PCT Application WO 03/068926 to Dow Global Technologies, filed February 13, 2003, entitled, "Over-Expression of Extremozyme Genes in *Pseudomonas* and Closely Related Bacteria" describes an expression system in which pseudomonads, specifically *P. fluorescens,* can be used as host cells for the production of extremozyme enzymes. These enzymes are typically ancient, found in prokaryotes, eukaryotes including fungi, yeast, lichen, protists and protozoa, algae and mosses, tardigrades and fish. The patent discloses that enzymes can be derived from certain extremophilic fungi and yeast, but are typically derived from extremophilic bacteria.

PCT publication No. WO 03/089455 to Dow Global Technologies, filed April 22, 2003, entitled "Low-Cost Production of Peptides" describes a method of producing small peptides, primarily antimicrobial peptides, as concatameric precursors in Pseudomonads, specifically *P. fluorescens.*

PCT publication No. WO 04/005221 to Dow Global Technologies, entitled "Benzoate and Antranilate Inducible Promoters" provides novel benzoate- or anthranilate-inducible promoters from *P. fluorescens,* as well as novel tandem promoters, variants and improved mutants thereof, that are useful for commercial prokaryotic fermentation systems.

U.S. Patent No. 5,232,840 to Monsanto Co. describes the use of novel ribosomal binding sites to enhance expression of certain proteins in prokaryotic cells. In one example, the cells are used to express porcine growth hormone in several organisms, including *E. coli, P. fluorescens,* and *P. putida.* The data shows that *P. fluorescens* is less efficient at expressing the growth hormone when compared to *E. coli.* In contrast, when expressing a bacterial protein, *P. fluorescens* is much more effective at protein production than *E. coli* under comparable conditions. In fact, *P. fluorescens* cells described in this patent produce several-fold more bacterially-derived β-galactosidase than *E. coli* (compare table 4 to tables 1 and 2).

While progress has been made in the production of proteins of commercial interest, a strong need remains to improve the capability and production level of recombinant mammalian, and in particular human, proteins.

### SUMMARY OF THE INVENTION

It has been discovered that *Pseudomonas fluorescens* is a superior organism for the production of recombinant proteins, and in particular recombinant mammalian proteins, such as recombinant human proteins. Based on these discoveries, the present invention provides a process of producing recombinant mammalian or mammalian-derived proteins in *P. fluorescens.* Accordingly, in a first aspect the invention provides a method for producing a recombinant mammalian protein in a *Pseudomonas fluorescens* host cell comprising: transforming a host cell with a nucleic acid encoding a recombinant mammalian protein; growing the cell under conditions that allow expression of the recombinant mammalian protein, wherein the recombinant mammalian protein is present in the host cell in a soluble or insoluble form; and wherein the recombinant mammalian protein is operably linked to a periplasmic secretion leader coding sequence which targets the protein to the periplasm; and wherein the protein is expressed at an increased level when compared to an expression level of the protein under substantially comparable conditions in an *E. coli* expression system.

In addition, the invention provides *P. fluorescens* transformed to produce recombinant mammalian, including human, proteins. Accordingly, in a second aspect the invention provides a *Pseudomonas fluorescens* cell comprising a nucleic acid encoding a recombinant human peptide, wherein the recombinant human peptide is operably linked to a periplasmic secretion leader coding sequence which targets the protein to the periplasm.

In one embodiment, the invention provides a process of producing a mammalian protein in a *P. fluorescens* organism in which the protein is produced at a higher level or concentration per cell or per liter of fermentation reaction than in an *E. coli* organism under comparable conditions. In yet another embodiment, the invention provides a process of producing mammalian proteins in an *P. fluorescens* organism in a batch culture which produces higher amounts of protein per liter than a corresponding batch of recombinant *E*. *coli* organisms.

Comparable conditions or substantially comparable conditions particularly refers to expression of recombinant protein using the same operably linked transcriptional promoter and ribosomal binding site in different organisms, and using the same initial induction conditions. Comparable conditions can further include using the same vector and associated regulatory elements, including, but not limited to, enhancer sequences, termination sequences, and origin or replication sequences. Comparable conditions can also include the same total volume of cell fermentation reaction. Comparable conditions can also include the same concentration of total cells per liter of reaction. In one embodiment, the conditions also include total induction times (before measurement) that are similar or the same. However, in another embodiment, the induction times can vary depending on the organism. Specifically, *P. fluorescens* has a capacity for increased growth time over *E. coli* without reducing protein production, such that protein production can be measured in *P. fluorescens* at a time point at which *E. coli* cells are largely silent. One way to measure the comparable conditions is to compare the percentage of recombinant protein per total cell protein. The comparable conditions also do not require identical media for growth. The media can be adjusted to ensure optimal production for the individual organisms.

In another embodiment, the invention provides a process for producing recombinant mammalian proteins by producing the proteins in a *P. fluorescens* organism and isolating the produced protein. In one sub-embodiment, the process includes substantially purifying the protein. In one embodiment, the protein is derived from a human protein, or is humanized.

The invention also provides a method of producing recombinant mammalian protein in *P. fluorescens* in at least the following embodiments:
(i) the production of recombinant mammalian, including human, proteins present in the cell in a range of between 1 and 75 percent total cell protein (%tcp), or in particular, at least greater than approximately 5% tcp, 10% tcp, at least 15% top, at least 20% tcp or more;
(ii) the production of recombinant mammalian, including human, proteins that are soluble and present in the cytoplasm of the cell in a range of between 1 and 75 %tcp or in particular, at least greater than approximately 5% tcp, 10% tcp, at least 15% tcp, at least 20% tcp or more;
(iii) the production of recombinant mammalian, including human, proteins that are insoluble in the cytoplasm of the cell, in a range of between 1 and 75 % tcp or in particular, at least greater than approximately 5% tcp, 10% tcp, at least 15% tcp, at least 20% tcp or more;
(iv) the production of recombinant mammalian, including human, proteins that are soluble in the periplasm of the cell in a range of between 1 and 75 % tcp or in particular, at least greater than approximately 5% tcp, 10% tcp, at least 15% tcp, at least 20% tcp or more;
(v) the production of recombinant mammalian, including human, proteins that are insoluble in the periplasm in a range of between 1 and 75 % tcp or in particular, at least greater than approximately 5% tcp, 10% tcp, at least 15% tcp, at least 20% tcp or more;
(vi) the production or recombinant mammalian, including human, proteins in the cell in a range of between 1 and 75 %tcp, or particularly at least greater than approximately 5% tcp, 10% tcp, at least 15% tcp, at least 20% tcp or more, when grown at a cell density of at least 40g/L;
(vii) the production of recombinant mammalian, including human, proteins present in the cell in an active form;
(viii) the production of multi-subunit recombinant mammalian, including human, proteins in active form;
(ix) the production of recombinant mammalian, including human, proteins that are then isolated and purified; and
(x) the production of recombinant mammalian, including human, proteins that are renatured.

In one embodiment, the recombinant mammalian protein is selected from the group consisting of a multi-subunit protein, a blood carrier protein, an enzyme, a full length antibody, an antibody fragment, or a transcriptional factor.

The *Pseudomonas fluorescens* cell of the second aspect of the invention includes:
(i) *Pseudomonas fluorescens* organisms that are transformed to produce recombinant mammalian, including human, proteins at a higher level or concentration than a corresponding *E. coli* organism when grown under substantially corresponding conditions;
(ii) *Pseudomonas fluorescens* organisms that are transformed to produce recombinant mammalian, including human, proteins and peptides that are present in the cell in a range of between 1 and 75% tcp or in particular, at least greater than approximately 5% tcp, 10% tcp, at least 15% tcp, at least 20% tcp or more;
(iii) *Pseudomonas fluorescens* organisms that are transformed to produce recombinant mammalian, including human, proteins that are present in the cell in active form;
(iv) *Pseudomonas fluorescens* organisms that are transformed to produce recombinant mammalian, including human, proteins that are soluble in the cytoplasm of the cell in a range of between 1 and 75 % tcp or in particular, at least greater than approximately 5% tcp, 10% tcp, at least 15% tcp, at least 20% tcp or more;
(v) *Pseudomonas fluorescens* organisms that are transformed to produce recombinant mammalian, including human, proteins that are insoluble in the cytoplasm of the cell in a range of between 1 and 75 % tcp or in particular, at least greater than approximately 5% tcp, 10% tcp, at least 15% tcp, at least 20% tcp or more;
(vi) *Pseudomonas fluorescens* organisms that are transformed to produce recombinant mammalian, including human, proteins that are soluble in the periplasm of the cell in a range of between 1 and 75 % tcp or in particular, at least greater than approximately 5% tcp, 10% tcp, at least 15% tcp, at least 20% tcp or more;
(vii) *Pseudomonas fluorescens* organisms that are transformed to produce recombinant mammalian, including human, proteins that are insoluble in the periplasm of the cell in a range of between 1 and 75 % tcp or in particular, at least greater than approximately 5% tcp, 10% tcp, at least 15% tcp, at least 20% tcp or more;
(viii) *Pseudomonas fluorescens* organisms that are transformed to produce multi-subunit recombinant mammalian, including human, proteins;
(ix) *Pseudomonas fluorescens* organisms that are transformed to produce multi-subunit recombinant mammalian, including human, proteins present in the cell in active form.

In an alternative embodiment, a *Pseudomonas* organisms and closely related bacteria other than *fluorescens* are used as host cells in this invention, as described in more detail below. The host cell will be selected generally from the genus *Pseudomonas* and specifically from a nonpathogenic *Pseudomonas* species. Likewise, any *Pseudomonas fluorescens* strain can be used that accomplishes the desired inventive goal, including but not limited to strain MB101, or a strain that is modified to include at least one host-cell-expressible, inserted copy of at least one Lac repressor protein-encoding lacI transgene, such as MB214 and MB217. The *Pseudomonas* organism can also optionally be genetically modified to add or delete one or more genes to improve performance, processing, or other characteristics.

In one embodiment, the *Pseudomonas* organism is transformed with a nucleic acid encoding a recombinant mammalian protein selected from the group consisting of a multi-subunit protein, a blood carrier protein, an enzyme, a full length antibody, an antibody fragment, or a transcriptional factor. In one embodiment, the *P. fluorescens* organism expresses a recombinant mammalian protein selected from the group consisting of a multi-subunit protein, a blood carrier protein, an enzyme, a full length antibody, an antibody fragment, or a transcriptional factor.

The expressed recombinant mammalian or human protein will typically have a mass of at least about lkD, and up to about 100, 200, 300, 400 or 500 kD, often between about 10kD and about 100kD, and usually greater than about 30kD.

### BRIEF DESCRIPTION OF THE FIGURES

**Figure 1** is a graph showing hu-γ-IFN purified from the soluble fraction of *P. fluorescens* samples displays activity comparable to a commercially available standard.
**Figure 2** is a picture of an ELISA showing the activity of purified Gal13 in *P. fluorescens* and *E. coli.*
**Figure 3** represents human growth hormone expression constructs. The amino acid sequence of human growth hormone lacking its native secretion signal sequence is shown in A. Plasmid constructs for expression in *P. fluorescens* (pDOW2400) and *E. coli* (412-001.hGH) are shown in B.
**Figure 4** is a picture of an SDS-PAGE analysis of soluble and insoluble fractions of hGH expressed in *P. fluorescens* and *E. coli.* The time post-induction is denoted by 10, 124, I48, 0 or 3. The large arrows indicate the position of the 21 kDa hGH protein.
**Figure 5** shows an SDS-PAGE analysis of the expression of γ-IFN in *E. coli* versus *P. fluoresens* cells. Soluble (S) and insoluble (I) fractions of samples taken at 0, 3, 24 and 48 hours post-induction (10, etc.) were resolved. *E. coli* expressed γ-IFN is shown in panel A, *P. fluorescens* expressed γ-IFN is shown in panel B. 5uL of A575-20 samples were loaded onto a 10% Bis-Tris NuPAGE gel and resolved in IX MES. Arrows indicate the position of the recombinant protein. Western analyses are shown in panels C (*E. coli*) and D (*P. fluorescens*)*.*

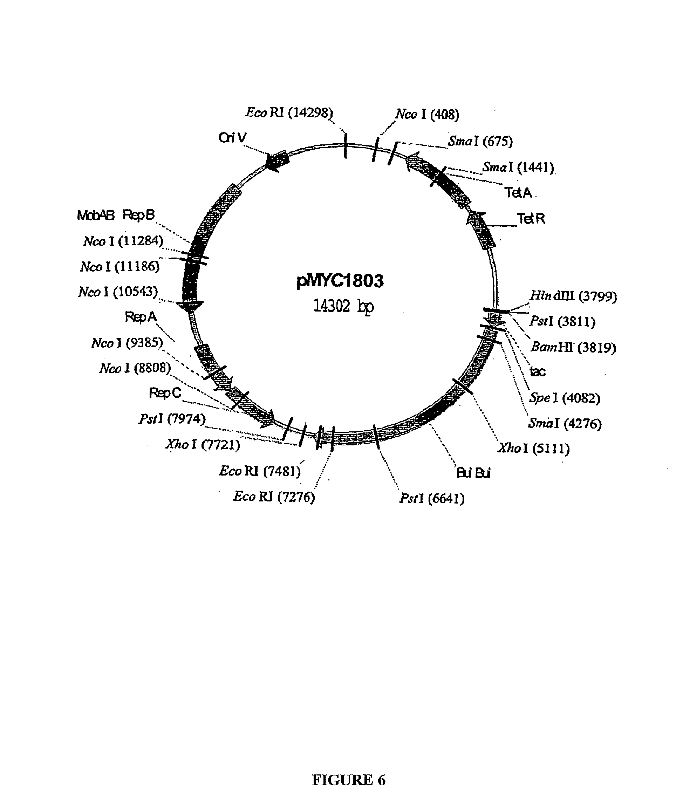

**Figure 6** shows the replacement of the BuiBui toxin gene with the BGI gene at the *Spe*I and *Xho*I sites of pMYC1803.

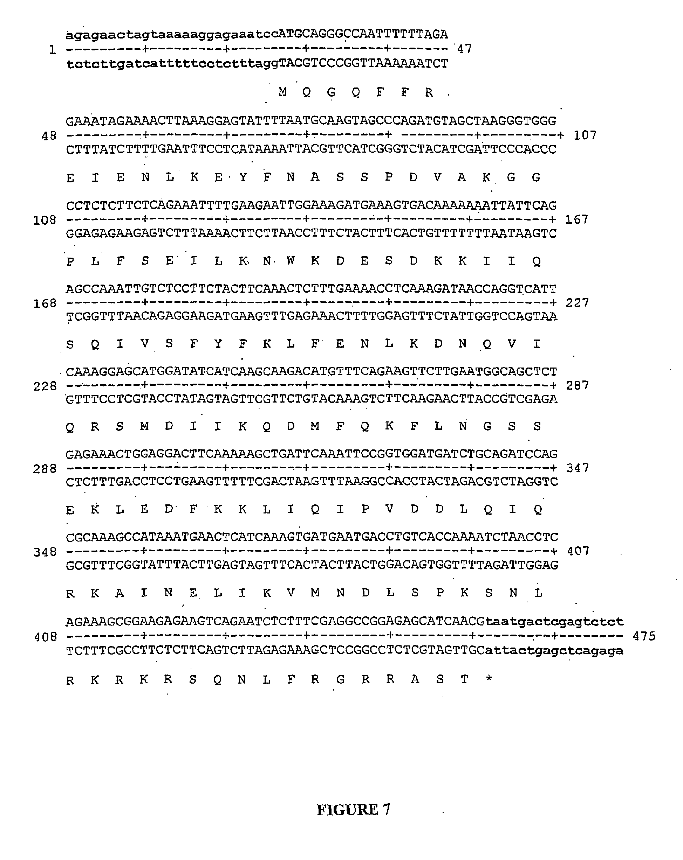

**Figure 7** shows that all the transformants selected had the desired interferon insert, as verified by sequencing the inserted DNA.
**Figure 8** represents the nucleotide sequence for the phosphate binding protein-gal2 single chain antibody fusion protein.
**Figure 9** represents the amino acid sequence for the phosphate binding protein-gal2 single chain antibody fusion protein.
**Figure 10** represents the nucleotide sequence for the phosphate binding protein-human growth hormone fusion protein.
**Figure 11** represents the amino acid sequence for the phosphate binding protein-human growth hormone fusion protein.

### DETAILED DESCRIPTION OF THE INVENTION

### Process for producing recombinant mammalian proteins

The invention provides processes and transformed *Pseudomonas fluorescens* organisms that produce recombinant mammalian proteins.

In one embodiment, the invention provides a process for producing recombinant mammalian proteins by producing the proteins in a *P. fluorescens* organism and isolating the produced protein. The protein can be isolated after expression by techniques known in the art, including, but not limited to, affinity chromatography, ion-exchange chromatography, antibody affinity, size-exclusion, or any other method that eliminates a substantial portion of the cellular debris from the protein. In one sub-embodiment, the process provides a substantially purified protein. The isolated protein can have activity similar to that of the native protein that it is derived from. The protein can be isolated in a correctly folded state or conformation, approximating that of the native protein, or can be further renatured or modified to put it into a correctly folded conformation. In one sub-embodiment, the protein is derived from a human protein, or is humanized. A "humanized" protein is typically a chimeric mammalian-type protein which is partially comprised of a human-derived protein sequence. Humanization is particularly useful in antibody production and the development of humanized antibodies has been extensively described, for example in U.S. Patent No. 6,800,738.

In one embodiment, expression of the protein by the host cell is followed by isolation of the protein. In another embodiment, the protein of peptide is purified. In an alternative embodiment, the protein is purified following isolation of the protein. Optionally the isolated or purified protein can be renatured or refolded in order to produce active proteins.

The first aspect of the invention provides a process of producing a mammalian protein in a *P. fluorescens* organism in which the protein is produced at a higher level or concentration than in an *E. coli* organism. The suitability of *P. fluorescens* organisms for high level production of mammalian proteins was unexpected based on the lack of success in producing such proteins in these organisms in the prior art. The present inventors have found that these organisms are indeed capable of high levels of production of mammalian proteins, and typically express protein in higher yield or at higher levels than *E. coli* when tested in corresponding assays. In another embodiment, the invention provides a process of producing mammalian proteins in an *P. fluorescens* organism in a batch culture which produces higher amounts of protein per liter than a corresponding batch of recombinant *E. coli* organisms.

In some embodiments, processes are provided that include producing recombinant mammalian, including human, multi-subunit proteins in active form in *P. fluorescens*; producing recombinant mammalian blood carrier proteins, including human blood carrier proteins such as transferrin and albumin in *P. fluorescens*; producing recombinant mammalian enzymes, including recombinant mammalian enzymes in active form in *P. fluorescens*; producing antibodies and antibody fragments, including single chain antibodies and Fab fragments in *P. fluorescens;* and producing recombinant mammalian, including human, transcriptional factors in *P.fluorescens.*

In one embodiment, the recombinant mammalian protein is produced as a multimer, or in a concatameric precursor, for example, in the form of at least two small peptide (1-15 amino acids) units in tandem. In an alternative embodiment, the recombinant mammalian protein is not produced as a multimer, or in concatameric precursors, but instead is produced as a single chain polypeptide.

### Screening of biomolecules

As described herein *P.fluorescens* organisms may be used in a process of screening libraries of mammalian biomolecules to identify at least one that exhibits a desired activity or property. The *P. fluorescens* cells can be transformed with a number of mammalian derived nucleic acids for which testing is desired, producing a library of transformed host cells. Upon expression,
polypeptides encoded by at least some of the nucleic acids are produced for testing either in cytoplasm or following recovery from the cell.
Examples of activities and properties for which testing may be performed include: polypeptide expression level; polypeptide stability; and biocatalytic activities and properties. Illustrative examples of biocatalytic activities and properties include: enzymatic activities; protein interactions/binding; protein stabilization; substrate usage; product formation; reaction conditions, such as pH, salinity, or reaction temperature; biocatalytic parameters for a given catalyzed reaction, such as Km and Vmax; and stability behavior, such as thermostability and biocatalyst half-life. The test results obtained may be used to selected library member(s) for further development.

### Protein expression

A key aspect of this invention is the expression of high levels of recombinant mammalian, for example human, proteins in a range of between 1 and 75 percent total cell protein (%tcp) by expression in *P. fluorescens* organisms. The expressed proteins are soluble or insoluble while in the *P.fluorescens* cell. Such high levels of soluble or insoluble recombinant mammalian proteins can be an improvement over previously known mammalian protein expression systems. In particular, high levels of recovered mammalian proteins in large scale fermentation reactions are not typically possible with known techniques.

The method of the first aspect of the invention allows expression levels of mammalian proteins that exceed those found in *E. coli* expression systems. In one embodiment, the concentration of recombinant protein in each cell is higher than that found in *E. coli* in comparative assays. In one embodiment, the level of recombinant protein as compared to total cell protein measured in the *P. fluorescens* expression system is higher than that of the same recombinant protein expressed in *E. coli.* In another embodiment, the level or amount of soluble protein in the *P. fluorescens* expression system described herein is higher than the level or amount of soluble recombinant protein in a comparable *E. coli* expression system. In another embodiment, the total amount of active protein is higher than the amount derived from an *E. coli* expression system. In a separate embodiment, the level of recombinant active protein as compared to total cell protein measured in the *P. fluorescens* expression system is higher than that of the same recombinant protein expressed in *E. coli.* In one embodiment, the level, concentration, or amount of protein expressed in *P. fluorescens* is at least 2x, at least 3x, at least 4x, at least 5x, at least 6x, at least 7x, at least 8x, at least 9x, at least 10x, or more the level, concentration, or amount of recombinant protein expressed in *E*. *coli* in comparable assays.

One of the benefits of *P. fluorescens* as an expression system is that the cells can be grown in large scale cultures without negatively impacting their capacity for protein production. This capacity exceeds that found in other bacterial systems, such as *E. coli.* In another embodiment, the process includes producing mammalian proteins in batch cultures in which the recombinant protein is produced at a higher total level in *P. fluorescens* than in *E*. *coli* batch cultures. In yet another embodiment, the invention provides a process of producing mammalian proteins in an *P. fluorescens* organism in a batch culture which produces higher amounts of protein per liter than a corresponding batch of recombinant *E. coli* organisms.

The invention generally provides processes and transformed *P. fluorescens* organisms that afford expression levels of 1-75% total cell protein (tcp) of soluble or insoluble recombinant mammalian proteins. The recombinant mammalian proteins expressed in the cell can be expressed in an active form. In other embodiments, the *P. fluorescens* provides at least 1, 5, 10, 15, 20, 25, 30, 40, 50, 55, 60, 65, 70, or 75 % tcp of recombinant mammalian proteins.

These proteins can be soluble, and when soluble, can be present in the cytoplasm or periplasm of the cell during production. Soluble proteins are readily released from the cell by methods including, but not limited to, rupturing of the cell membrane by pressure (i.e. the "French" press method), or by lysozyme degradation of the cell membrane. Cells can typically also be lysed using detergents, such as non-ionic detergents. Proteins that are soluble can be further stabilized by adjusting components of the buffer, such as buffer pH, salt concentrations, or additional protein components (for example, in multi-subunit complexes). The soluble proteins can be isolated or purified from other protein and cellular debris by, for example, centrifugation and/or chromatography such as size exclusion, anion or cation exchange, or affinity chromatography.

The proteins can also be insoluble. Insoluble proteins are typically found in inclusion bodies in the cytoplasm, but are also often in the periplasm. Not all insoluble proteins are in inclusion bodies, and can also be found in membrane aggregates, as small protein aggregates or in any other insoluble form in the cytoplasm or periplasm. Insoluble proteins can typically be renatured using, for example, reducing agents such as urea or guanidine hydrochloride. Insoluble proteins or protein aggregates can be isolated, for example, by centrifugation and/or chromatography such as size exclusion chromatography. Proteins in insoluble aggregates can typically be separated by solubilization of the aggregates using, for example, micelles or reverse micelles as described in Vinogradov, et al. (2003) Anal Biochem. 15;320(2):234-8.

In a particular embodiment, the *Pseudomonas* host cell can have a recombinant mammalian peptide, polypeptide, protein, or fragment thereof expression level of at least 1% tcp and a cell density of at least 40 g/L, when grown (i.e. within a temperature range of about 4° C to about 55° C, inclusive) in a mineral salts medium. In a particular embodiment, the expression system will have a recombinant protein of peptide, including recombinant mammalian protein, expression level of at least 5% tcp and a cell density of at least 40 g/L, when grown (i.e. within a temperature range of about 4° C to about 55° C, inclusive) in a mineral salts medium at a fermentation scale of at least 10 Liters.

Expression levels can be measured by standard techniques known in the art. In one embodiment, the amount of protein (in grams) is compared to the amount in grams of total cell protein in a given sample. In another embodiment, the measurement is a level of recombinant protein per liter. In another embodiment, the level or amount can be measured as compared to a known standard, such as a BSA control. The level or amount of recombinant protein can be measured, for example, by analyzing the light absorbtion of a purified protein, by measuring an affinity of an marker for the protein (such as an antibody affinity) and comparing that to a known standard, or by measuring the level of activity compared to a known standard (such as a known amount of purified, active protein).

It has been found that, in certain situations, no additional disulfide-bond-promoting conditions or agents are required in order to recover disulfide-bond-containing target polypeptides in active, soluble form, when a *Pseudomonas fluorescens* bacteria is used as the expression host cell. Therefore, in one embodiment, the transgenic peptide, polypeptide, protein, or fragment contains at least one intramolecular disulfide bond in its native state. In other embodiments, the protein can contain up to 2, 4, 6, 8, 10, 12, 14, 16, 18, or 20 or more disulfide bonds in its native state.

In some embodiments, the protein is expressed or found in the periplasm of the cell during production before purification or isolation. The protein is secreted into the periplasm by being fused to an appropriate signal secretion sequence. In one embodiment, the signal sequence is a signal sequence that is native to the *P. fluorescens* genome. In specific embodiments, the signal sequence is a phosphate binding protein, a Lys-Arg-Orn binding protein (LAObp or KRObp) secretion signal peptide, an Outer Membrane Porin E (OpreE) secretion signal peptide, an azurin secretion signal peptide, an iron (III) binding protein (Fe(III)bp) secretion signal peptide, or a lipoprotein B (LprB) secretion signal peptide.

In one embodiment, the recombinant peptide, polypeptide, protein, or fragment thereof has a folded intramolecular conformation in its active state. *P. fluorescens* typically produce mammalian proteins more efficiently in the correctly folded conformation. In one embodiment, more than 50% of the expressed, transgenic peptide, polypeptide, protein, or fragment thereof produced can be produced as single peptides, polypeptides, proteins, or fragments thereof in soluble, active form or insoluble, but renaturable form in the cytoplasm or periplasm. In another embodiment about 60% , 70%, 75%, 80%, 85%, 90%, 95% of the expressed protein is obtained in or can be renatured into active form.

### Definitions

Throughout this specification, the term "protein" is used to include any amino acid concatamers or polymers. The terms "polypeptide," "peptide" and "protein" are used interchangeably and include amino acid polymers in which one or more amino acid residue is an artificial chemical analogue of a corresponding naturally occurring amino acid, as well as to naturally occurring amino acid polymers.

The term "isolated" refers to nucleic acid, protein, or peptide that is substantially or essentially free from other material components which normally accompany it as found in its native state when in a cell, for example, other cellular components.

The term "purified" or "substantially purified" is used to mean that the protein is separated from other cell components and is separated from other proteins and peptides found in the cell that are not in a native complex with the protein. In particular embodiments, the purified proteins are of a purity approved for therapeutic or veterinary used as defined by standard cGMP guidelines or approved by the FDA.

The term "percent total cell protein" ("tcp") means the amount of protein in the host cell as a percentage of aggregate cellular protein. Alternatively, the term means a measure of the fraction of total cell protein that represents the relative amount of a given protein expressed by the cell.

The term "operably attached" refers to any configuration in which the transcriptional and any translational regulatory elements are covalently attached to the encoding sequence in such disposition(s), relative to the coding sequence, that in and by action of the host cell, the regulatory elements can direct the expression of the coding sequence.

As used herein, the term "mammal" is meant to include or designate any animal in the class Mammalia including human or non-human mammals, such as, but not limited, to porcine, ovine, bovine, rodents, ungulates, pigs, swine, sheep, lambs, goats, cattle, deer, mules, horses, monkeys, apes, dogs, cats, rats, and mice.

As used herein, the term "recombinant mammalian protein" or peptide is meant to include proteins derived from a native mammalian protein sequence or derived or generated from a native mammalian nucleic acid sequence. Such recombinant proteins can be produced from nucleic acid sequence substantially corresponding to native mammalian mRNA or substantially corresponding cDNA, or fragements thereof. The sequence can be adjusted accordingly based on specific host cell codon usage as known in the art.

The phrase "substantially corresponding" in the context of two nucleic acids or polypeptides refers to the residues in the two sequences that have at least 50%, 60%, 70%, 80%, 90% or higher identity when aligned for maximum correspondence over a domain of the protein, as measured using an algorithms known in the art. Optimal alignment of sequences for comparison can be conducted, e.g., by algorithms known in the art (e.g. Smith & Waterman (1981) Adv. Appl. Math. 2:482; Needleman & Wunsch (1970) J. Mol. Biol. 48:443; Pearson & Lipman (1988) Proc. Nat'l. Acad. Sci. USA 85:2444; Altschul et al. (1990) J. Mol. Biol. 215:403-410 (BLAST)), by computerized implementations of these algorithms (GAP, BESTFIT, FASTA, and TFASTA in the Wisconsin Genetics Software Package, Genetics Computer Group, 575 Science Dr., Madison, Wis.), or by inspection.. Software for performing BLAST analyses is publicly available through the National Center for Biotechnology Information (http://www.ncbi.nlm.nih.gov/).

The term "fragment" means a portion or partial sequence of a nucleotide, protein, or peptide sequence.

As used herein, the term "soluble" means that the protein is not precipitated by centrifugation at between approximately 5,000x and 20,000x gravity when spun for 10-30 minutes in a buffer under physiological conditions. Soluble proteins are not part of an inclusion body or other precipitated mass.

As used herein, the term "insoluble" means that the protein that can be precipitated by centrifugation at between 5,000x and 20,000x gravity when spun for 10-30 minutes in a buffer under physiological conditions. Insoluble proteins can be part of an includion body or other precipitated mass.

The term "inclusion body" is meant to include any intracellular body contained within a cell wherein an aggregate of proteins have been sequestered.

As used herein, the term "homologous" means either i) a protein that has an amino acid sequence that at least 70, 75, 80, 85, 90, 95, or 98% similar to the sequence of a given original protein and that retains a desired function of the original protein or ii) a nucleic acid that has a sequence that is at least 70, 75, 80, 5, 90, 95, or 98% similar to the sequence of a given nucleic acid and that retains a desired function of the original nucleic acid sequence. In all of the embodiments of this invention and disclosure, any disclosed protein, peptide or nucleic acid can be substituted with a homologous or substantially homologous protein, peptide or nucleic acid that retains a desired function. In all of the embodiments of this invention and disclosure, when any nucleic acid is disclosed, it should be assumed that the invention also includes all nucleic acids that hybridize to the disclosed nucleic acid.

In one non-limiting embodiment, the non-identical amino acid sequence of the homologous polypeptide can be amino acids that are members of any one of the 15 conservative or semi-conservative groups shown in -Table 1.

**TABLE 1. Similar Amino Acid Substitution Groups**

| Conservative Groups (8) | Semi-Conservative Groups (7) |
|---|---|
| Arg, Lys | Arg, Lys, His |
| Asp, Glu | Asn, Asp, Glu, Gln |
| Asn, Gln | |
| Ile, Leu, Val | Ile, Leu, Val, Met, Phe |
| Ala, Gly | Ala, Gly, Pro, Ser, Thr |
| Ser, Thr | Ser, Thr, Tyr |
| Phe, Tyr | Phe, Trp, Tyr |
| Cys (non-cystine), Ser | Cys (non-cystine), Ser, Thr |

### Types of Mammalian Proteins Produced

In general, the recombinant mammalian protein can be any mammalian protein of which an amino acid sequence is known or any putative mammalian or mammalian-derived protein for which an amino acid sequence is deduced. The proteins can be selected from the group consisting of a multi-subunit protein, a blood carrier protein, an enzyme, a full length antibody, an antibody fragment, or a transcriptional factor.

The amino acid sequence of the protein can be altered to adjust for desired qualities, such as to ensure certain types of interactions. The sequence can, for example, be adjusted to reduce immunoreactivity, or to increase absorbtion, reduce excretion, or otherwise enhance bioavailability in an organism such as a mammal. The amino acid sequence of the protein can thus be adjusted to ensure certain post-translational modifications or protein conformations.

In one embodiment, the mammalian protein is a chemokin or cytokine. In another embodiment, the mammalian proteins is one of the following proteins: IL-1, IL-1α, IL-1β, IL-2, IL-3, IL-4, IL-5, IL-6, IL-7, IL-8, IL-9, IL-10, IL-11, IL-12, IL-12elasti, IL-13, IL-15, IL-16, IL-18, IL-18BPa, IL-23, IL-24, VIP, erythropoietin, GM-CSF, G-CSF, M-CSF, platelet derived growth factor (PDGF), MSF, FLT-3 ligand, EGF, fibroblast growth factor (FGF; e.g., aFGF (FGF-1), bFGF (FGF-2), FGF-3, FGF-4, FGF-5, FGF-6, or FGF-7), insulin-like growth factors (e.g., IGF-1, IGF-2); tumor necrosis factors (e.g., TNF, Lymphotoxin), nerve growth factors (e.g., NGF), vascular endothelial growth factor (VEGF); interferons (e.g., IFN-α, IFN-β, IFN-y); leukemia inhibitory factor (LIF); ciliary neurotrophic factor (CNTF); oncostatin M; stem cell factor (SCF); transforming growth factors (e.g., TGF-α, TGF-β1, TGF-β1, TGF-β1); TNF superfamily (e.g., LIGHT/TNFSF14, STALL-1/TNFSF13B (BLy5, BAFF, THANK), TNFalpha/TNFSF2 and TWEAK/TNFSF12); or chemokines (BCA-1/BLC-1, BRAK/Kec, CXCL16, CXCR3, ENA-78/LIX, Eotaxin-1, Eotaxin-2/MPIF-2, Exodus-2/SLC, Fractalkine/Neurotactin, GROalpha/MGSA, HCC-1, I-TAC, Lymphotactin/ATAC/SCM, MCP-1/MCAF, MCP-3, MCP-4, MDC/STCP-1/ABCD-1, MIP-1α, MIP-1β, MIP-2α/GROβ, MIP-3α/Exodus/LARC, MIP-3α/Exodus-3/ELC, MIP-4/PARC/DC-CK1, PF-4, RANTES, SDF1α, TARC, or TECK).

Alternatively, the protein is not a chemokine or cytokine. In another embodiment, the mammalian protein is not one of the following proteins: IL-1, IL-1α, IL-1β, IL-2, IL-3, IL-4, IL-5, IL-6, IL-7, IL-8, IL-9, IL-10, IL-11, IL-12, IL-12elasti, IL-13, IL-15, IL-16, IL-18, IL-18BPa, IL-23, IL-24, VIP, erythropoietin, GM-CSF, G-CSF, M-CSF, platelet derived growth factor (PDGF), MSF, FLT-3 ligand, EGF, fibroblast growth factor (FGF; e.g., aFGF (FGF-1), bFGF (FGF-2), FGF-3, FGF-4, FGF-5, FGF-6, or FGF-7), insulin-like growth factors (e.g., IGF-1, IGF-2); tumor necrosis factors (e.g., TNF, Lymphotoxin), nerve growth factors (e.g., NGF), vascular endothelial growth factor (VEGF); interferons (e.g., IFN-α, IFN-β, IFN-γ); leukemia inhibitory factor (LIF); ciliary neurotrophic factor (CNTF); oncostatin M; stem cell factor (SCF); transforming growth factors (e.g., TGF-α, TGF-β1, TGF-β1, TGF-β1); TNF superfamily (e.g., LIGHT/TNFSF14, STALL-1/TNFSF13B (BLy5, BAFF, THANK), TNFalpha/TNFSF2 and TWEAK/TNFSF12); or chemokines (BCA-1/BLC-1, BRAK/Kec, CXCL16, CXCR3, ENA-78/LIX, Eotaxin-1, Eotaxin-2/MPIF-2, Exodus-2/SLC, Fractalkine/Neurotactin, GROalpha/MGSA, HCC-1, I-TAC, Lymphotactin/ATAC/SCM, MCP-1/MCAF, MCP-3, MCP-4, MDC/STCP-1/ABCD-1, MIP-1α, MIP-1β, MIP-2α/GROβ, MIP-3α/Exodus/LARC, MIP-3α/Exodus-3/ELC, MIP-4/PARC/DC-CK1, PF-4, RANTES, SDF1α, TARC, or TECK). In one embodiment, the protein is not a porcine protein, particularly not a porcine growth factor.

As yet a further embodiment of the present disclosure, the recombinant mammalian proteins, their fragments or other derivatives, or analogs thereof, can be antibodies. These antibodies can be, for example, polyclonal or monoclonal antibodies. This aspect of the present invention also includes chimeric, single chain, and humanized antibodies, as well as Fab fragments, or the product of an Fab expression library.

### Production of Multi-subunit Proteins

In one embodiment of the present invention, the production of recombinant mammalian multi-subunit proteins by a host cell of the species *Pseudomonas fluorescens* is provided. In another embodiment, a host cell of the *Pseudomonas fluorescens* species is provided that has been transformed to express a recombinant mammalian multi-subunit protein. In one embodiment, multisubunit proteins, including recombinant mammalian or human proteins, are expressed in a *Pseudomonas fluorescens* host cell. In one embodiment, expression of the multi-subunit protein by the host cell is followed by isolation of the multi-subunit protein. In another embodiment, the multi-subunit protein of peptide is purified. The protein can be assembled by the cell before purification or isolation, or further assembly can be undertaken during or after isolation or purification. Optionally, the protein or any portion thereof can be renatured or refolded to produce active proteins.

Any of a variety of vectors and expression systems can be used to express the multi-subunit protein in the host cell. The multi-subunits can be located on a single vector, optionally operably linked to different promoters, optionally in a polycistronic sequence. Each subunit can also be on different vectors. Multiple vectors can be used. Each subunit can be under the control of one or more selection markers. Regulatory elements can be included on the vector, including periplasmic secretion signal sequences, internal ribosome entry sites, activator sequences, promoters, and termination signals.

In one embodiment, multisubunit proteins are expressed in *Pseudomonas fluorescens* using expression systems with auxotrophic selection markers as disclosed in U.S. Application No. 10/994138 to Dow Global Technologies filed November 19, 2004, wherein the control of each nucleic acid encoding a subunit is under the control of an auxotrophic selection marker. Multisubunit proteins that can be expressed include homomeric and heteromeric proteins. The multisubunit proteins may include two or more subunits, that may be the same or different. For example, the protein may be a homomeric protein comprising 2, 3, 4, 5, 6, 7, 8, 9, 10, 11, 12 or more subunits. The protein also may be a heteromeric protein including 2, 3, 4, 5, 6, 7, 8, 9, 10, 11, 12, or more subunits.

Exemplary multisubunit mammalian proteins include: receptors including ion channel receptors; signaling proteins such as kinases, GTPases, ATPases; transmembrane proteins; extracellular matrix proteins including chondroitin; collagen; immunomodulators including MHC proteins, full chain antibodies, and antibody fragments; enzymes including RNA polymerases, and DNA polymerases; and membrane proteins.

### Production of Blood Proteins

In one embodiment of the present invention, the production of recombinant mammalian blood proteins is provided. In one embodiment, expression of the blood protein by the host cell is followed by isolation of the blood protein. In another embodiment, the blood protein is purified. In another embodiment, following isolation of the blood protein, the blood protein is purified. Optionally, the protein can be renatured or refolded to produce active protein. In general, a recombinant blood protein of this invention is produced by transforming a suitable host cell, such as a *P. fluorescens* host cell, with a nucleic acid construct encoding the blood protein, culturing the transformed host cell under conditions appropriate for expression, and optionally isolating, or isolating and purifying the recombinant blood protein expressed by the cell.

In another embodiment, a host cell of the *Pseudomonas fluorescens* species is provided that has been transformed to express a recombinant mammalian blood protein with an vector containing appropriate genes and regulatory elements for expression of the blood protein of interest is provided.

The blood proteins that can be expressed include, but are not limited to: carrier proteins, such as albumin, including human albumin (Seq ID No. 1, Table 2) and bovine albumin; transferrin, including human transferrin (Seq ID No. 2, Table 2), bovine transferrin, rat transferrin, recombinant transferrin, recombinant transferrin half-molecules, recombinant transferrin half-molecules having altered properties; haptoglobin; fibrinogen and other coagulation factors; complement components; immunoglobulins; enzyme inhibitors; precursors of substances such as angiotensin and bradykinin; insulin; endothelin; globulin including alpha, beta, and gamma-globulin; and other types of proteins, peptides, and fragments thereof found primarily in the blood of mammals. The amino acid sequences for numerous blood proteins have been reported (see, S. S. Baldwin (1993) Comp. Biochem Physiol. 106b: 203-218), including the amino acid sequence for human serum albumin (Lawn, L.M., et al. (1981) Nucleic Acids Research 9:22; pp 6103-6114) and human serum transferrin (Yang, F. el al. (1984) Proc. Natl. Acad. Sci. USA 81; pp. 2752-2756).

In a specific embodiment, the production of albumin in *P. fluorescens,* is provided, comprising transforming a *P. fluorescens* host cell with an expression vector containing a nucleic acid sequence or sequences and regulatory elements for expression of albumin, culturing the host cell under conditions suitable for expression of the albumin, and recovering the albumin expressed by *P. fluorescens.* According to this embodiment, the albumin expressed is selected from the group consisting of human albumin, bovine albumin, rabbit albumin, chicken albumin, rat albumin, and mouse albumin. In another embodiment, albumin can be fused to a therapeutically active polypeptide, which can be of mammalian or non-mammalian origin.

In a further specific embodiment, the production of a transferrin in *P. fluorescens* is provided, comprising transforming a *P. fluorescens* host cell with an expression vector containing nucleic acid and regulatory elements for expression of the transferrin, culturing the host cell under conditions suitable for expression of the transferring. In another embodiment, following expression of the transferrin, and, in one embodiment, isolating the protein. In a further embodiment, the transferrin can be purified following isolation. The transferrin expressed is selected from the group consisting of human serum transferrin, glycosylated human transferrin, non-glycosylated human transferrin, the N-terminal half-molecule of human transferrin, bovine transferrin, rat transferrin, mouse transferrin, primate transferrin, recombinant transferrin, recombinant transferrin half-molecules, recombinant transferrin half-molecules having altered properties, transferrin polynucleotides, transferrin polypeptides encoded by transferrin polypeptides, transferrin polypeptides, transferrin antibodies, transferrin fragments, and transferrin fused to a therapeutically active polypeptide.

In yet another specific embodiment, the production of a globulin in *P. fluorescens* is provided, comprising transforming a *P. fluorescens* host cell with an expression vector containing nucleic acid and regulatory elements for expression of the globulin, culturing the host cell under conditions suitable for expression of the globulin and optionally isolating the protein. In a further embodiment, following expression, the globulin is isolated and purified from the host cell. The globulin expressed is selected from the group consisting of human globulin, bovine globulin, rabbit globulin, rat globulin, mouse globulin, sheep globulin, monkey globulin, steroid-binding globulins, and globulin fused to a therapeutically active polypeptide.

In a further embodiment, the production of an insulin in *P. fluorescens* is provided, comprising transforming a *P. fluorescens* host cell with an expression vector containing nucleic acid and regulatory elements for expression of the insulin, culturing the host cell under conditions suitable for expression of the insulin and optionally isolatig the protein. In a further embodiment, the insulin can be isolated and purified following production of the insulin by the host cell. The insulin expressed is selected from the group consisting of human insulin, bovine insulin, mouse insulin, rat insulin, porcine insulin, monkey insulin, and insulin fused to a therapeutically active polypeptide. The accession number for human insulin genes is J00265, and for synthetic human insulin gene the accession number is J02547.

Full-length DNA for production of recombinant blood proteins or truncated DNA encoding either the amino-terminal or carboxy-terminal lobe of blood proteins or a portion thereof can be obtained from available sources or can be synthesized according to the known sequences by standard procedures.

**Table 2. Sequences of blood proteins expressed by the system of the present disclosure.**

| | | |
|---|---|---|
| Amino Acid Sequence of human serum albumin | Seq. ID. No: 1 | |
| | | (Lawn, et al. (1981) Nuc. Ac. Rsch. 9(22):6103-6114) |
| Amino Acid | Seq. ID. No: 2 | |
| Sequence of transferrin | | |
| | | (Strausberg (2002) PNAS, 99:16899-16903) |
| cDNA Sequence of Human Insulin | Seq. ID. No: 3 | |

### Production of Enzymes

In one embodiment of the present invention, the production of recombinant mammalian enzymes or co-factors by a host cell of the species *Pseudomonas fluorescens* is provided. In another embodiment, a host cell of the Pseudomonas species is provided that has been transformed to express a recombinant mammalian enzyme or co-factor.

The enzymes and co-factors expressed in this embodiment include but are not limited to aldolases, amine oxidases, amino acid oxidases, aspartases, B₁₂ dependent enzymes, carboxypeptidases, carboxyesterases, carboxylyases, chemotrypsin, CoA requiring enzymes, cyanohydrin synthetases, cystathione synthases, decarboxylases, dehydrogenases, alcohol dehydrogenases, dehydratases, diaphorases, dioxygenases, enoate reductases, epoxide hydrases, fumerases, galactose oxidases, glucose isomerases, glucose oxidases, glycosyltrasferases, methyltransferases, nitrile hydrases, nucleoside phosphorylases, oxidoreductases, oxynitilases, peptidases, glycosyltrasferases, peroxidases, and enzymes fused to a therapeutically active polypeptide.

In another embodiment, the enzyme can be a mesophilic enzyme polypeptide, for example one that is desirable for human and/or veterinary therapeutic and/or diagnostic use. Examples of such therapeutic mesophilic enzymes include, e.g., tissue plasminogen activator; urokinase, reptilase, streptokinase; catalase, superoxide dismutase; DNAse, amino acid hydrolases (e.g., asparaginase, amidohydrolases); carboxypeptidases; proteases, trypsin, pepsin, chymotrypsin, papain, bromelain, collagenase; neuraminidase; lactase, maltase, sucrase, and arabinofuranosidases.

Yet another embodiment provides for the production of recombinant enzyme replacements in *P. fluorescens* cells by transforming a *P. fluorescens* host cell with an expression vector containing nucleic acids and regulatory elements for xpression of recombinant enzyme replacements, and culturing the cell under conditions suitable for expression of the recombinant enzyme replacements. The recombinant enzyme replacements expressed in the host cell is selected from the group consisting of Algasidase beta, Laronidase, and recombinant enzyme replacements fused to a therapeutically active polypeptide.

### Production of Mammalian Antibodies and Antibody Fragments

In one embodiment of the present invention, the production of recombinant mammalian single chain, Fab fragments and/or full chain antibodies or fragments or portions thereof by a host cell of the species *P. fluorescens* is provided. In one embodiment, following expression of the protein, the protein can be isolated and optionally purified. Optionally, the protein can be renatured to produce an active protein. The antibody or antibody fragments are optionally linked to a secretion signal sequence for targeting in the cell during production.

In another embodiment, a host cell of the *Pseudomonas fluorescens* species is provided that has been transformed to express a recombinant mammalian single chain, Fab fragments and/or full chain antibodies or fragments or portions thereof.

In one embodiment, the *P. fluorescens* cell can produces a single chain antibody or fragments or portions thereof. A single-chain antibody can include the antigen-binding regions of antibodies on a single stably-folded polypeptide chain. Single-chain antibodies are of smaller size than classical immunoglobulins but can retain the antigen-specific binding properties of antibodies. It is disclosed that single chain antibodies can be used for therapeutics, such as "naked" single-chain antibodies, bi-specific antibody binders, radioconjugates or as fusions with effector domains, diagnostics, such as tumor imaging or in vivo or ex vivo cancer marker assays, research tools, such as protein purification and detection, including identification and characterization of novel therapeutic targets, antibody microarrays, display technologies and/or vehicles for gene or drug delivery.

In another embodiment, the *P. fluorescens* cell produces Fab fragments or portions thereof. Fab fragments can be a piece of a particular antibody. The Fab fragment can contain the antigen binding site. The Fab fragment can contains 2 chains : a light chain and a heavy chain fragment. These fragments can be linked via alinker or a disulfide bond.

In other embodiments of the present invention, full chain antibodies can be expressed in *P. fluorescens,* and other *Pseudomonas* species. An intact antibody containing the Fc region can be more resistant against degradation and clearance *in vivo,* thereby having longer biological half life in circulation. Such antibodies can be used as a therapeutic agent for diseases requiring sustained therapies.

In one embodiment, a process for producing a functional antibody or fragment thereof in *Pseudomonas* species is provided by providing an expression vector that contains separate cistronic or polycistronic sequences. The separate cistron expression vector can contain a first promoter-cistron pair for expression of an immunoglobulin light chain and a second promoter-cistron pair for expression of an immunoglobulin heavy chain, such that expression of the light chain and heavy chain are independently regulated by separate promoters. Each cistron within the expression cassette polynucleotide can include a translation initiation region (TIR) operably linked to the nucleic acid sequence coding for the light chain or heavy chain of the full length antibody. In one embodiment, the TIR sequences can be manipulated to provide different translational strength combinations for light and heavy chains. In an alternative embodiment, a heavy chain coding sequence can be located on the same plasmid as a light chain coding sequence. In an alternative embodiment, the heavy and light chain sequences are found in a polycistronic sequence within a single plasmid, or coded into the genome of the host.

In another embodiment, a process is provided for producing a functional antibody or fragment thereof in a host cell transformed with two separate translational units respectively encoding the light and heavy chains of the antibody. In one embodiment the process includes: a) culturing the host cell under suitable conditions so that the light chain and heavy chain are expressed in a sequential fashion, thereby temporally separating the production of the light and heavy chains; and b) allowing the assembly of the light and heavy chains to form the functional antibody or fragment thereof.

In further embodiment, the *Pseudomonas fluorescens* expression system can express human therapeutic single chain, Fab fragments or full chain antibodies or portions thereof, including, but not limited to Fab, Fab', F(ab')₂, F(ab')₂-leucine zipper, Fv, dsFv, anti-CD18 antibody, chimeric antibodies, human antibodies, humanized antibodies, or those described in the Table 3 below.

**Table 3 - Antibodies and Antibody Fragments.**

| Antibody | Target Antigen | Product Type | Isotype | Indication |
|---|---|---|---|---|
| 5G1.1 | Complement (C5) | Humanised | IgG | Rheumatoid Arthritis |
| 5G1.1 | Complement (C5) | Humanised | IgG | SLE |
| 5G1.1 | Complement (C5) | Humanised | IgG | Nephritis |
| 5G1.1-SC | Complement (C5) | Humanised | ScFv | Cardiopulmanory Bypass |
| 5G1.1-SC | Complement (C5) | Humanised | ScFv | Myocardial Infarction |
| 5G1.1-SC | Complement (C5) | Humanised | ScFv | Angioplasty |
| ABX-CBL | CBL | Human | | GvHD |
| ABX-CBL | CD147 | Murine | IgG | Allograft rejection |
| ABX-IL8 | IL-8 | Human | IgG2 | Psoriasis |
| AD-159 | gp120 | Humanised | | HIV |
| AD-439 | gp120 | Humanised | | HIV |
| Antegren | VLA-4 | Humanised | IgG | Multiple Sclerosis |
| Anti-CD 11a | CD11a | Humanised | IgG1 | Psoriasis |
| Anti-CD 18 | CD18 | Humanised | Fab'2 | Myocardial infarction |
| Anti-LFAl | CD18 | Murine | Fab'2 | Allograft rejection |
| Anti-VEGF | VEGF | Humanised | IgG1 | Cancer (general |
| Antova | CD40L | Humanised | IgG | Allograft rejection |
| Antova | CD40L | Humanised | IgG | SLE |
| BEC2 | anti-Id | Murine | IgG | Lung |
| BIRR-1 | ICAM-1 | Murine | IgG2a | Stroke |
| BTI-322 | CD2 | Rat | IgG | GvHD |
| C225 | EGFR | Chimeric | IgG | Head+Neck |
| CAT-152 | TGF-beta 2 | Human | | Glaucoma Surgery |
| CDP571 | TNF-alpha | Humanised | IgG4 | Crohn's |
| CDP571 | TNF-alpha | Humanised | IgG4 | Rheumatoid Arthritis |
| CDP850 | E-selectin | Humanised | | Psoriasis |
| Corsevin M | Fact VII | Chimeric | | Anticoagulant |
| D2E7 | TNF-alpha | Human | | Rheumatoid Arthritis |
| Herceptin | Her2/neu | Humanised | IgG1 | Metastatic Breast |
| HNK20 | F gp | Murine | IgA | RSV |
| Hu23F2G | CD11/18 | Humanised | | Multiple Sclerosis |
| Hu23F2G | CD11/18 | Humanised | IgG | Stroke |
| IC14 | CD14 | --- | | Toxic shock |
| ICM3 | ICAM-3 | Humanised | | Psoriasis |
| IDEC-114 | CD80 | Primatised | | Psoriasis |
| IDEC-131 | CD40L | Humanised | | SLE |
| IDEC-131 | CD40L | Humanised | | Multiple Sclerosis |
| IDEC-151 | CD4 | Primatised | IgG1 | Rheumatoid Arthritis |
| IDEC-152 | CD23 | Primatised | | Asthma/Allergy |
| Infliximab | TNF-alpha | Chimeric | IgG1 | Rheumatoid Arthritis |
| Infliximab | TNF-alpha | Chimeric | IgG1 | Crohn's |
| LDP-01 | beta2-integrin | Humanised | IgG | Stroke |
| LDP-01 | beta2-integrin | Humanised | IgG | Allograft rejection |
| LDP-02 | alpha4beta7 | Humanised | | Ulcerative Colitis |
| LDP-03 / Campath1H | CD52 | Humanised | IgG1 | CLL |
| Lym-1 | HLA DR | Chimeric | | NHL |
| LympoCide | CD22 | Humanised | | NHL |
| MAK-195F | TNF alpha | Murine | Fab'2 | Toxic shock |
| MDX-33 | CD64 (FcR) | Human | | Autoimmune haematogical disorders |
| MDX-CD4 | CD4 | Human | IgG | Rheumatoid Arthritis |
| MEDI-500 | TCR alpha beta | Murine | IgM | GvHD |
| MEDI-507 | CD2 | Humanised | | Psoriasis |
| MEDI-507 | CD2 | Humanised | | GvHD |
| OKT4A | CD4 | Humanised | IgG | Allograft rejection |
| OrthoClone OKT4A | CD4 | Humanised | IgG | Autoimmune disease |
| Orthoclone/ anti-CD3 OKT3 | CD3 | Murine | mIgG2 a | Allograft rejection |
| Ostavir | Hep B | Human | | Hep B |
| OvaRex | CA 125 | Murine | | Ovarian |
| Panorex 17-1A | EpCAM | Murine | IgG2a | Colorectal |
| PRO542 | gp120 | Humanised | | HIV |
| Protovir | CMV | Humanised | IgG1 | CMV |
| RepPro/Abc iximab | gpIIbIIIa | Chimeric | Fab | Complications of coronary angioplasty |
| rhuMab-E25 | IgE | Humanised | IgG1 | Asthma/Allergy |
| Rituxan | CD20 | Chimeric | IgG1 | NHL |
| SB-240563 | IL5 | Humanised | | Asthma/Allergy |
| SB-240683 | IL-4 | Humanised | | Asthma/Allergy |
| SCH55700 | IL-5 | Humanised | | Asthma/Allergy |
| Simulect | CD25 | Chimeric | IgG1 | Allograft rejection |
| SMART a-CD3 | CD3 | Humanised | | Autoimmune disease |
| SMART a-CD3 | CD3 | Humanised | | Allograft rejection |
| SMART a-CD3 | CD3 | Humanised | IgG | Psoriasis |
| SMART M195 | CD33 | Humanised | IgG | AML |
| SMART 1D10 | HLA | ---- | | NHL |
| Synagis | F gp | Humanised | IgG1 | RSV (Paediatric) |
| Vitaxin | VNRintegrin | Humanised | | Sarcoma |
| Zenapax | CD25 | Humanised | IgG1 | Allograft rejection |

### Production of Transcriptional Factors

In one embodiment of the present invention, the production of recombinant mammalian transcription factors by a host cell of the species *Pseudomonas fluorescens* is provided. In one embodiment, following expression of the protein, the protein can be isolated. In another embodiment, the protein can be purified. Optionally, the protein can be renatured to produce an active protein. In another embodiment, a host cell of the *Pseudomonas fluorescens* species is provided that has been transformed to express a recombinant mammalian transcription factor.

Transcription factors suitable for insertion into the expression systems of the present invention include those of the helix turn helix family and members of the Pac family, as well as other transcription factor families known in the art. Members of these families suitable for use with the present invention include mammalian and mammalian homologs and analogs of: transcriptional regulators; transcription factors of the of the ASNC family such as ASNC_trans_reg, putative transcriptional regulators; bacterial regulatory proteins of the luxR family; bacterial regulatory helix-turn-helix transcription factors; bacterial regulatory proteins of the arsR family; transcription factors of the helix-turn-helix domain, especially the rpiR family; bacterial regulatory protein transcription factors, bacterial regulatory helix-turn-helix transcription factors; DNA binding domain transcription factors; MarR family of transcription factors; the ROK family of transcription factors; the MerR family of regulatory proteins; arginine repressor transcription factors; firmicute transcriptional factors; ferric uptake regulatory transcription factors; sigma transcription factors; response regulatory receiver transcription factors; tryptophan RNA-binding attenuator protein transcription factors; putative sugar-binding domain transcription factors; PRD domain transcription factors; nitrogen regulatory protein transcription factors; negative regulators of genetic competence, such as MecA; negative transcriptional regulator transcription factors; bacterial transcriptional regulator transcription factors; glycerol-3 -phosphate responsive transcription factors; iron dependent repressor transcription factors; and numerous species specific transcriptional regulator transcription factors.

Transcriptional factors expressed by *Pseudomonas fluorescens* species can be utilized for diagnostic, therapeutic, and investigational applications.

### Vector Preparation

### Polynucleotides

The recombinant mammalian proteins and peptides can be expressed from polynucleotides in which the target polypeptide coding sequence is operably attached to transcription and translation regulatory elements forming a functional gene from which the host cell can express the protein. The coding sequence can be a native coding sequence for the target polypeptide, if available, but can also be a coding sequence that has been selected, improved, or optimized for use in the selected expression host cell: for example, by synthesizing the gene to reflect the codon use bias of a *Pseudomonas* species such as *P. fluorescens.* The gene(s) that result will have been constructed within or will be inserted into one or more vector, which will then be transformed into the expression host cell. Nucleic acid or a polynucleotide said to be provided in an "expressible form" means nucleic acid or a polynucleotide that contains at least one gene that can be expressed by the selected bacterial expression host cell.

### Regulatory Elements

The regulatory elements used herein can be operably attached to the target recombinant mammalian protein encoding gene. The coding sequence of the protein-encoding gene used herein can contain, in addition to the mature polypeptide coding sequence and transcription-regulatory elements, further encoding elements, e.g., one or more of coding sequences for peptide tags, pre-peptides, pro-peptides, pre-pro-peptides, or other commonly utilized encoding elements known in the art, excluding secretion signal peptides functional in the selected expression host cell.

The term "operably attached," as used herein, refers to any configuration in which the transcriptional and any translational regulatory elements are covalently attached to the coding sequence in such disposition(s), relative to the coding sequence, that the regulatory elements can direct the expression of the coding sequence. In one embodiment, the regulatory elements will be part of a whole gene before undergoing transformation into a host cell; however, in other embodiments the regulatory elements are part of another gene, which can be part of the host genome or can be part of a genome of another organism, or can be derived therefrom.

### Promoters and Accessory Elements

The promoters used in accordance with the present invention may be constitutive promoters or regulated promoters. Common examples of useful regulated promoters include those of the family derived from the lac promoter (i.e. the lacZ promoter), especially the tac and trc promoters described in U.S. Patent No. 4,551,433 to DeBoer, as well as Ptac16, Ptacl7, PtacII, PlacUV5, and the T7lac promoter.

Common examples of non-lac-type promoters useful in expression systems according to the present invention include, e.g., those listed in Table 4.

**Table 4. Examples of non-lac Promoters**

| Promoter | Inducer |
|---|---|
| λP_{R} | High temperature |
| λP_{L} | High temperature |
| Pm | Alkyl- or halo-benzoates |
| Pu | Alkyl- or halo-toluenes |
| Psal | Salicylates |

See, e.g.: J. Sanchez-Romero & V. De Lorenzo (1999) Manual of Industrial Microbiology and Biotechnology (A. Demain & J. Davies, eds.) pp.460-74 (ASM Press, Washington, D.C.); H. Schweizer (2001) Current Opinion in Biotechnology 12:439-445; and R. Slater & R. Williams (2000) Molecular Biology and Biotechnology (J. Walker & R. Rapley, eds.) pp.125-54. A promoter having the nucleotide sequence of a promoter native to the selected bacterial host cell may also be used to control expression of the transgene encoding the target polypeptide, e.g, a *Pseudomonas* anthranilate or benzoate operon promoter (Pant, Pben). Tandem promoters may also be used in which more than one promoter is covalently attached to another, whether the same or different in sequence, e.g., a Pant-Pben tandem promoter (interpromoter hybrid) or a Plac-Plac tandem promoter.

Regulated promoters utilize promoter regulatory proteins in order to control transcription of the gene of which the promoter is a part. Where a regulated promoter is used herein, a corresponding promoter regulatory protein will also be part of an expression system according to the present invention. Examples of promoter regulatory proteins include: activator proteins, e.g., *E. coli* catabolite activator protein, MalT protein; AraC family transcriptional activators; repressor proteins, e.g., *E. coli* LacI proteins; and dual-fuction regulatory proteins, e.g., *E. coli* NagC protein. Many regulated-promoter/promoter-regulatory-protein pairs are known in the art.

Promoter regulatory proteins interact with an effector compound, i.e. a compound that reversibly or irreversibly associates with the regulatory protein so as to enable the protein to either release or bind to at least one DNA transcription regulatory region of the gene that is under the control of the promoter, thereby permitting or blocking the action of a transcriptase enzyme in initiating transcription of the gene. Effector compounds are classified as either inducers or co-repressors, and these compounds include native effector compounds and gratuitous inducer compounds. Many regulated-promoter/promoter-regulatory-protein/ effector-compound trios are known in the art. Although an effector compound can be used throughout the cell culture or fermentation, in one embodiment in which a regulated promoter is used, after growth of a desired quantity or density of host cell biomass, an appropriate effector compound is added to the culture in order directly or indirectly result in expression of the desired target gene(s).

By way of example, where a lac family promoter is utilized, a *lacI* gene can also be present in the system. The *lacI* gene, which is (normally) a constitutively expressed gene, encodes the Lac repressor protein (LacI protein) which binds to the lac operator of these promoters. Thus, where a lac family promoter is utilized, the *lαcI* gene can also be included and expressed in the expression system. In the case of the lac promoter family members, e.g., the tac promoter, the effector compound is an inducer, such as a gratuitous inducer like IPTG (isopropyl-β-D-1-thiogalactopyranoside, also called "isopropylthiogalactoside").

### Other Elements

Other regulatory elements can be included in an expression construct. Such elements include, but are not limited to, for example, transcriptional enhancer sequences, translational enhancer sequences, other promoters, activators, translational start and stop signals, transcription terminators, cistronic regulators, polycistronic regulators, tag sequences, such as nucleotide sequence "tags" and "tag" peptide coding sequences, which facilitates identification, separation, purification, or isolation of an expressed polypeptide.

At a minimum, a protein-encoding gene according to the present invention can include, in addition to the mammalian protein coding sequence, the following regulatory elements operably linked thereto: a promoter, a ribosome binding site (RBS), a transcription terminator, translational start and stop signals. Useful RBSs can be obtained from any of the species useful as host cells in expression systems, such as from the selected host cell. Many specific and a variety of consensus RBSs are known, e.g., those described in and referenced by D. Frishman et al. (1999) Gene 234(2):257-65; and B.E. Suzek et al. (2001) Bioinformatics 17(12):1123-30. In addition, either native or synthetic RBSs may be used, e.g., those described in: EP 0207459 (synthetic RBSs); O. Ikehata et al. (1989) Eur. J. Biochem. 181(3):563-70 (native RBS sequence of AAGGAAG). Further examples of methods, vectors, and translation and transcription elements, and other elements useful in the present invention are described in, e.g.: U.S. Patent Nos. 5,055,294 and 5,128,130 to Gilroy et al.; 5,281,532 to Rammler et al.; 4,695,455 and 4,861,595 to Barnes et al.; 4,755,465 to Gray et al.; and 5,169,760 to Wilcox.

### Vectors

Transcription of the DNA encoding the enzymes of the present invention by *Pseudomonas* is increased by inserting an enhancer sequence into the vector or plasmid. Typical enhancers are cis-acting elements of DNA, usually about from 10 to 300 bp in size that act on the promoter to increase its transcription. Examples include various *Pseudomonas* enhancers, as described elsewhere herein.

Generally, the recombinant expression vectors will include origins of replication and selectable markers permitting transformation of the *Pseudomonas* host cell, e.g., the antibiotic-free resistance genes of *P. fluorescens,* and a promoter derived from a highly-expressed gene to direct transcription of a downstream structural sequence. Such promoters can be derived from operons encoding the enzymes such as 3-phosphoglycerate kinase (PGK), acid phosphatase, or heat shock proteins, among others. The heterologous structural sequence is assembled in appropriate phase with translation initiation and termination sequences, and, in one embodiment, a leader sequence capable of directing secretion of the translated enzyme. Optionally, the heterologous sequence can encode a fusion enzyme including an N-terminal identification peptide imparting desired characteristics, e.g., stabilization or simplified purification of expressed recombinant product.

Useful expression vectors for use with *P. fluorescens* in expressing enzymes are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host.

Vectors are known in the art as useful for expressing recombinant proteins in host cells, and any of these may be used for expressing the genes according to the present invention. Such vectors include, e.g., plasmids, cosmids, and phage expression vectors. Examples of useful plasmid vectors include, but are not limited to, the expression plasmids pBBR1MCS, pDSK519, pKT240, pML122, pPS10, RK2, RK6, pRO1600, and RSF1010. Other examples of such useful vectors include those described by, e.g.: N. Hayase (1994) Appl. Envir. Microbiol. 60(9):3336-42; A.A. Lushnikov et al. (1985) Basic Life Sci. 30:657-62; S. Graupner & W. Wackernagel (2000) Biomolec. Eng. 17(1):11-16.; H.P. Schweizer (2001) Curr. Opin. Biotech. 12(5):439-45; M. Bagdasarian & K.N. Timmis (1982) Curr. Topics Microbiol. Immunol. 96:47-67; T. Ishii et al. (1994) FEMS Microbiol. Lett. 116(3):307-13; I.N. Olekhnovich & Y.K. Fomichev (1994) Gene 140(1):63-65; M. Tsuda & T. Nakazawa (1993) Gene 136(1-2):257-62; C. Nieto et al. (1990) Gene 87(1):145-49; J.D. Jones & N. Gutterson (1987) Gene 61(3):299-306; M. Bagdasarian et al. (1981) Gene 16(1-3):237-47; H.P. Schweizer et al. (2001) Genet. Eng. (NY) 23:69-81; P. Mukhopadhyay et al. (1990) J. Bact. 172(1):477-80; D.O. Wood et al. (1981) J. Bact. 145(3):1448-51; and R. Holtwick et al. (2001) Microbiology 147(Pt 2):337-44.

Further examples of expression vectors that can be useful in *Pseudomonas* host cells include those listed in Table 5 as derived from the indicated replicons.

**Table 5. Some Examples of Useful Expression Vectors**

| Replicon | Vector(s) |
|---|---|
| pPS10 | pCN39, pCN51 |
| RSF1010 | pKT261-3 |
| | pMMB66EH |
| | pEB8 |
| | pPLGN1 |
| | pMYC1050 |
| RK2/RP1 | pRK415 |
| | pJB653 |
| pRO1600 | pUCP |
| | pBSP |

The expression plasmid, RSF1010, is described, e.g., by F. Heffron et al. (1975) Proc. Nat'l Acad. Sci. USA 72(9):3623-27, and by K. Nagahari & K. Sakaguchi (1978) J. Bact. 133(3):1527-29. Plasmid RSF1010 and derivatives thereof are particularly useful vectors in the present invention. Exemplary, useful derivatives of RSF1010, which are known in the art, include, e.g., pKT212, pKT214, pKT231 and related plasmids, and pMYC1050 and related plasmids (see, e.g., U.S. Patent Nos. 5,527,883 and 5,840,554 to Thompson et al.), such as, e.g., pMYC1803. Plasmid pMYC1803 is derived from the RSF1010-based plasmid pTJS260 (see U.S. Patent No. 5,169,760 to Wilcox), which carries a regulated tetracycline resistance marker and the replication and mobilization loci from the RSF1010 plasmid. Other exemplary useful vectors include those described in U.S. Patent No. 4,680,264 to Puhler et al.

In a one embodiment, an expression plasmid is used as the expression vector. In another embodiment, RSF1010 or a derivative thereof is used as the expression vector. In still another embodiment, pMYC1050 or a derivative thereof, or pMYC1803 or a derivative thereof, is used as the expression vector.

The plasmid can be maintained in the host cell by use of a selection marker gene, also present in the plasmid. This may be an antibiotic resistance gene(s), in which case the corresponding antibiotic(s) will be added to the fermentation medium, or any other type of selection marker gene known as useful in the art, e.g., a prototrophy-restoring gene in which case the plasmid will be used in a host cell that is auxotrophic for the corresponding trait, e.g., a biocatalytic trait such as an amino acid biosynthesis or a nucleotide biosynthesis trait or a carbon source utilization trait.

Extensive sequence information required for molecular genetics and genetic engineering techniques is widely publicly available. Access to complete nucleotide sequences of mammalian, as well as human, genes, cDNA sequences, amino acid sequences and genomes can be obtained from GenBank at the URL address http://www.ncbi.nlm.nih.gov/Entrez. Additional information can also be obtained from GeneCards, an electronic encyclopedia integrating information about genes and their products and biomedical applications from the Weizmann Institute of Science Genome and Bioinformatics (http://bioinformatics.weizmann.ac.il/cards/), nucleotide sequence information can be also obtained from the EMBL Nucleotide Sequence Database ( http://www.ebi.ac.uk/embl/) or the DNA Databank or Japan (DDBJ, http://www.ddbj.nig.ac.jp/; additional sites for information on amino acid sequences include Georgetown's protein information resource website (http://www-nbrf.georgetown.edu/pir/) and Swiss-Prot (http://au.expasy.org/sprot/sprot-top.html).

### Transformation

Transformation of the *Pseudomonas* host cells with the vector(s) may be performed using any transformation methodology known in the art, and the bacterial host cells may be transformed as intact cells or as protoplasts (i.e. including cytoplasts). Exemplary transformation methodologies include poration methodologies, e.g., electroporation, protoplast fusion, bacterial conjugation, and divalent cation treatment, e.g., calcium chloride treatment or CaCl/Mg²⁺ treatment, or other well known methods in the art. See, e.g., Morrison (1977) J. Bact. 132:349-351; Clark-Curtiss & Curtiss (1983) Methods in Enzymology 101:347-362, Sambrook et al. (1989) Molecular Cloning, A Laboratory Manual (2nd ed.); Kriegler (1990) Gene Transfer and Expression: A Laboratory Manual; and Ausubel et al., eds. (1994) Current Protocols in Molecular Biology*.*

### Pseudomonas Organisms

While the invention herein is the use of *Pseudomonas fluorescens,* other *Pseudomonas* and closely related bacterial organisms which can be useful are also disclosed herein. *Pseudomonas* and closely related bacteria are generally part of the group defined as "Gram(-) Proteobacteria Subgroup 1" or "Gram-Negative Aerobic Rods and Cocci" (Buchanan and Gibbons (eds.) (1974) Bergey's Manual of Determinative Bacteriology, pp. 217-289).

**Table 6. "Gram-Negative Aerobic Rods and Cocci" (Bergey (1974))**

| | |
|---|---|
| Family I. Pseudomonadaceae | *Gluconobacter* |
| | *Pseudomonas* |
| | *Xanthomonas* |
| | *Zoogloea* |
| Family II. Azotobacteraceae | *Azomonas* |
| | *Azotobacter* |
| | *Beijerinckia* |
| | *Derxia* |
| Family III. Rhizobiaceae | *Agrobacterium* |
| | *Rhizobium* |
| Family IV. Methylomonadaceae | *Methylococcus* |
| | *Methylomonas* |
| Family V. Halobacteriaceae | *Halobacterium* |
| | *Halococcus* |
| Other Genera | *Acetobacter* |
| | *Alcaligenes* |
| | *Bordetella* |
| | *Brucella* |
| | *Francisella* |
| | *Thermus* |

"Gram-negative Proteobacteria Subgroup 1" also includes Proteobacteria that would be classified in this heading according to the criteria used in the classification. The heading also includes groups that were previously classified in this section but are no longer, such as the genera *Acidovorax, Brevundimonas, Burkholderia, Hydrogenophaga, Oceanimonas, Ralstonia, and Stenotrophomonas,* the genus *Sphingomonas* (and the genus *Blastomonas,* derived therefrom), which was created by regrouping organisms belonging to (and previously called species of) the genus *Xanthomonas, the* genus *Acidomonas,* which was created by regrouping organisms belonging to the genus *Acetobacter* as defined in Bergey (1974). In addition hosts can include cells from the genus *Pseudomonas* , *Pseudomonas enalia* (ATCC 14393), *Pseudomonas nigrifaciens* (ATCC 19375), and *Pseudomonas putrefaciens* (ATCC 8071), which have been reclassified respectively as *Alteromonas haloplanktis, Alteromonas nigrifaciens*, and *Alteromonas putrefaciens.* Similarly, e.g., *Pseudomonas acidovorans* (ATCC 15668) and *Pseudomonas testosteroni* (ATCC 11996) have since been reclassified as *Comamonas acidovorans* and *Comamonas testosteroni,* respectively; and *Pseudomonas nigrifaciens*(ATCC 19375) and *Pseudomonas piscicida* (ATCC 15057) have been reclassified respectively as *Pseudoalteromonas nigrifaciens* and *Pseudoalteromonas piscicida.* " Gram-negative Proteobacteria Subgroup 1" also includes Proteobacteria classified as belonging to any of the families: Pseudomonadaceae, Azotobacteraceae (now often called by the synonym, the "Azotobacter group" of Pseudomonadaceae), Rhizobiaceae, and Methylomonadaceae (now often called by the synonym, " Methylococcaceae"). Consequently, in addition to those genera otherwise described herein, further Proteobacterial genera falling within " Gram-negative Proteobacteria Subgroup 1" include: 1) Azotobacter group bacteria of the genus *Azorhizophilus*; 2) Pseudomonadaceae family bacteria of the genera *Cellvibrio, Oligella, and Teredinibacter*; 3) Rhizobiaceae family bacteria of the genera *Chelatobacter, Ensifer, Liberibacter* (also called *"Candidatus Liberibacter"),* and *Sinorhizobium*; and 4) Methylococcaceae family bacteria of the genera *Methylobacter, Methylocaldum, Methylomicrobium, Methylosarcina,* and *Methylosphaera.*

Also disclosed herein is a host cell selected from " Gram-negative Proteobacteria Subgroup 2." " Gram-negative Proteobacteria Subgroup 2" is defined as the group of Proteobacteria of the following genera (with the total numbers of catalog-listed, publicly-available, deposited strains thereof indicated in parenthesis, all deposited at ATCC, except as otherwise indicated): *Acidomonas* (2); *Acetobacter* (93); *Gluconobacter* (37); *Brevundimonas (23); Beijerinckia* (13); *Derxia* (2); *Brucella* (4); *Agrobacterium* (79); *Chelatobacter* (2) *Ensifer* (3); *Rhizobium* (144); *Sinorhizobium* (24); *Blastomonas* (1); *Sphingomonas* (27) *Alcaligenes* (88); *Bordetella* (43); *Burkholderia* (73); *Ralstonia* (33); *Acidovorax* (20) *Hydrogenophaga* (9); *Zoogloea* (9); *Methylobacter* (2); *Methylocaldum* (1 at NCIMB); *Methylococcus* (2); *Methylomicrobium* (2); *Methylomonas* (9); *Methylosarcina* (1) *Methylosphaera; Azomonas* (9); *Azorhizophilus* (5); *Azotobacter* (64); *Cellvibrio* (3); *Oligella* (5); *Pseudomonas* (1139); *Francisella* (4); *Xanthomonas* (229); *Stenotrophomonas* (50); *and Oceanimonas* (4).

Exemplary host cell species of " Gram-negative Proteobacteria Subgroup 2" include, but are not limited to the following bacteria (with the ATCC or other deposit numbers of exemplary strain(s) thereof shown in parenthesis): *Acidomonas methanolica* (ATCC 43581); *Acetobacter aceti* (ATCC 15973); *Gluconobacter oxydans* (ATCC 19357); *Brevundimonas diminuta* (ATCC 11568); *Beijerinckia indica* (ATCC 9039 and ATCC 19361); *Derxia gummosa* (ATCC 15994); *Brucella melitensis* (ATCC 23456), *Brucella abortus* (ATCC 23448); *Agrobacterium tumefaciens* (ATCC 23308), *Agrobacterium radiobacter* (ATCC 19358), *Agrobacterium rhizogenes* (ATCC 11325); *Chelatobacter heintzii* (ATCC 29600); *Ensifer adhaerens* (ATCC 33212); *Rhizobium leguminosarum* (ATCC 10004); *Sinorhizobium fredii* (ATCC 35423); *Blastomonas natatoria* (ATCC 35951); *Sphingomonas paucimobilis* (ATCC 29837); *Alcaligenes faecalis* (ATCC 8750); *Bordetella pertussis* (ATCC 9797); *Burkholderia cepacia* (ATCC 25416); *Ralstonia pickettii* (ATCC 27511); *Acidovorax facilis* (ATCC 11228); *Hydrogenophaga flava* (ATCC 33667); *Zoogloea ramigera* (ATCC 19544); *Methylobacter luteus* (ATCC 49878); *Methylocaldum gracile* (NCIMB 11912); *Methylococcus capsulatus* (ATCC 19069); *Methylomicrobium agile* (ATCC 35068); *Methylomonas methanica* (ATCC 35067); *Methylosarcina fibrata* (ATCC 700909); *Methylosphaera hansonii* (ACAM 549); *Azomonas agilis* (ATCC 7494); *Azorhizophilus paspali* (ATCC 23833); *Azotobacter chroococcum* (ATCC 9043); *Cellvibrio mixtus* (UQM 2601); *Oligella urethralis* (ATCC 17960); *Pseudomonas aeruginosa* (ATCC 10145), *Pseudomonas fluorescens* (ATCC 35858); *Francisella tularensis* (ATCC 6223); *Stenotrophomonas maltophilia* (ATCC 13637); *Xanthomonas campestris* (ATCC 33913); and *Oceanimonas doudoroffii* (ATCC 27123).

Also disclosed herein is a host cell selected from " Gram-negative Proteobacteria Subgroup 3." "Gram-negative Proteobacteria Subgroup 3" is defined as the group of Proteobacteria of the following genera: *Brevundimonas; Agrobacterium; Rhizobium; Sinorhizobium; Blastomonas; Sphingomonas; Alcaligenes; Burkholderia; Ralstonia; Acidovorax; Hydrogenophaga; Methylobacter; Methylocaldum; Methylococcus; Methylomicrobium; Methylomonas; Methylosarcina; Methylosphaera; Azomonas; Azorhizophilus; Azotobacter; Cellvibrio; Oligella; Pseudomonas; Teredinibacter; Francisella; Stenotrophomonas; Xanthomonas;* and *Oceanimonas.*

Also disclosed herein is a host cell is selected from "Gram-negative Proteobacteria Subgroup 4." "Gram-negative Proteobacteria Subgroup 4" is defined as the group of Proteobacteria of the following genera: *Brevundimonas; Blastomonas; Sphingomonas ; Burkholderia; Ralstonia; Acidovorax; Hydrogenophaga; Methylobacter; Methylocaldum; Methylococcus; Methylomicrobium; Methylomonas; Methylosarcina; Methylosphaera; Azomonas; Azorhizophilus; Azotobacter; Cellvibrio; Oligella; Pseudomonas; Teredinibacter; Francisella ; Stenotrophomonas; Xanthomonas;* and *Oceanimonas.*

Also disclosed herein, is a host cell selected from "Gram-negative Proteobacteria Subgroup 5." "Gram-negative Proteobacteria Subgroup 5" is defined as the group of Proteobacteria of the following genera: *Methylobacter; Methylocaldum; Methylococcus; Methylomicrobium; Methylomonas; Methylosarcina; Methylosphaera; Azomonas; Azorhizophilus; Azotobacter; Cellvibrio; Oligella; Pseudomonas; Teredinibacter; Francisella; Stenotrophomonas; Xanthomonas;* and *Oceanimonas.*

The host cell can be selected from "Gram-negative Proteobacteria Subgroup 6." "Gram-negative Proteobacteria Subgroup 6" is defined as the group of Proteobacteria of the following genera: *Brevundimonas; Blastomonas; Sphingomonas; Burkholderia; Ralstonia; Acidovorax; Hydrogenophaga; Azomonas; Azorhizophilus; Azotobacter; Cellvibrio; Oligella; Pseudomonas; Teredinibacter; Stenotrophomonas; Xanthomonas;* and *Oceanimonas.*

The host cell can be selected from "Gram-negative Proteobacteria Subgroup 7." "Gram-negative Proteobacteria Subgroup 7" is defined as the group of Proteobacteria of the following genera: Azomonas; *Azorhizophilus; Azotobacter; Cellvibrio; Oligella; Pseudomonas; Teredinibacter; Stenotrophomonas; Xanthomonas;* and *Oceanimonas.*

The host cell can be selected from "Gram-negative Proteobacteria Subgroup 8." "Gram-negative Proteobacteria Subgroup 8" is defined as the group of Proteobacteria of the following genera: *Brevundimonas; Blastomonas; Sphingomonas; Burkholderia; Ralstonia; Acidovorax; Hydrogenophaga; Pseudomonas; Stenotrophomonas; Xanthomonas;* and *Oceanimonas.*

The host cell can be selected from "Gram-negative Proteobacteria Subgroup 9." "Gram-negative Proteobacteria Subgroup 9" is defined as the group of Proteobacteria of the following genera: *Brevundimonas; Burkholderia; Ralstonia; Acidovorax; Hydrogenophaga; Pseudomonas; Stenotrophomonas;* and *Oceanimonas.*

The host cell can be selected from "Gram-negative Proteobacteria Subgroup 10." "Gram-negative Proteobacteria Subgroup 10" is defined as the group of Proteobacteria of the following genera: *Burkholderia; Ralstonia; Pseudomonas; Stenotrophomonas;* and *Xanthomonas.*

The host cell can be selected from "Gram-negative Proteobacteria Subgroup 11." "Gram-negative Proteobacteria Subgroup 11" is defined as the group of Proteobacteria of the genera: *Pseudomonas; Stenotrophomonas;* and *Xanthomonas.* The host cell can be selected from "Gram-negative Proteobacteria Subgroup 12." "Gram-negative Proteobacteria Subgroup 12" is defined as the group of Proteobacteria of the following genera: *Burkholderia; Ralstonia; Pseudomonas.* The host cell can be selected from "Gram-negative Proteobacteria Subgroup 13." "Gram-negative Proteobacteria Subgroup 13" is defined as the group of Proteobacteria of the following genera: *Burkholderia; Ralstonia; Pseudomonas;* and *Xanthomonas.* The host cell can be selected from "Gram-negative Proteobacteria Subgroup 14." "Gram-negative Proteobacteria Subgroup 14" is defined as the group of Proteobacteria of the following genera: *Pseudomonas* and *Xanthomonas.* The host cell can be selected from "Gram-negative Proteobacteria Subgroup 15." "Gram-negative Proteobacteria Subgroup 15" is defined as the group of Proteobacteria of the genus *Pseudomonas.*

The host cell can be selected from "Gram-negative Proteobacteria Subgroup 16." "Gram-negative Proteobacteria Subgroup 16" is defined as the group of Proteobacteria of the following *Pseudomonas* species (with the ATCC or other deposit numbers of exemplary strain(s) shown in parenthesis): *P. abietaniphila* (ATCC 700689); *P. aeruginosa* (ATCC 10145); *P. alcaligenes* (ATCC 14909); *P. anguilliseptica* (ATCC 33660); *P. citronellolis* (ATCC 13674); *P.flavescens* (ATCC 51555); *P. mendocina* (ATCC 25411); *P. nitroreducens* (ATCC 33634); *P. oleovorans* (ATCC 8062); *P. pseudoalcaligenes* (ATCC 17440); *P*. *resinovorans* (ATCC 14235); *P. straminea* (ATCC 33636); *P. agarici* (ATCC 25941); *P*. *alcaliphila; P. alginovora; P. andersonii; P. asplenii* (ATCC 23835); *P. azelaica* (ATCC 27162); *P. beijerinckii* (ATCC 19372); *P. borealis; P. boreopolis* (ATCC 33662); *P*. *brassicacearum; P. butanovora* (ATCC 43655); *P. cellulosa* (ATCC 55703); *P. aurantiaca* (ATCC 33663); *P. chlororaphis* (ATCC 9446, ATCC 13985, ATCC 17418, ATCC 17461); *P. fragi* (ATCC 4973); *P. lundensis* (ATCC 49968); *P. taetrolens* (ATCC 4683); *P. cissicola* (ATCC 33616); *P. coronafaciens; P. diterpeniphila; P. elongata* (ATCC 10144); *P. flectens* (ATCC 12775); *P. azotoformans; P. brenneri; P. cedrella; P. corrugata* (ATCC 29736); *P*. *extremorientalis; P. fluorescens* (ATCC 35858); *P. gessardii; P. libanensis; P. mandelii* (ATCC 700871); *P. marginalis* (ATCC 10844); P. *migulae; P. mucidolens* (ATCC 4685); *P*. *orientalis; P. rhodesiae; P. synxantha* (ATCC 9890); *P*. *tolaasii* (ATCC 33618); *P. veronii* (ATCC 700474); *P. frederiksbergensis; P. geniculata* (ATCC 19374); *P*. *gingeri; P. graminis; P. grimontii; P. halodenitrificans; P. halophila; P. hibiscicola* (ATCC 19867); *P*. *huttiensis* (ATCC 14670); *P*. *hydrogenovora; P. jessenii* (ATCC 700870); *P*. *kilonensis; P. lanceolata* (ATCC 14669); *P. lini; P. marginata* (ATCC 25417); *P. mephitica* (ATCC 33665); *P. denitrificans* (ATCC 19244); *P. pertucinogena* (ATCC 190); *P. pictorum* (ATCC 23328); *P. psychrophila; P. fulva* (ATCC 31418); *P. monteilii* (ATCC 700476); *P. mosselii; P. oryzihabitans* (ATCC 43272); *P. plecoglossicida* (ATCC 700383); *P. putida* (ATCC 12633); *P. reactans; P. spinosa* (ATCC 14606); *P. balearica; P. luteola* (ATCC 43273); *P*. *stutzeri* (ATCC 17588); *P*. *amygdali* (ATCC 33614); *P. avellanae* (ATCC 700331); *P*. *caricapapayae* (ATCC 33615); *P. cichorii* (ATCC 10857); *P. ficuserectae* (ATCC 35104); *P. fuscovaginae; P. meliae* (ATCC 33050); *P. syringae* (ATCC 19310); *P. viridiflava* (ATCC 13223); *P. thermocarboxydovorans* (ATCC 35961); *P. thermotolerans; P. thivervalensis; P. vancouverensis* (ATCC 700688); *P. wisconsinensis;* and *P*. *xiamenensis.*

The host cell can be selected from "Gram-negative Proteobacteria Subgroup 17." "Gram-negative Proteobacteria Subgroup 17" is defined as the group of Proteobacteria known in the art as the "fluorescent Pseudomonads" including those belonging, e.g., to the following *Pseudomonas* species: *P*. *azotoformans; P. brenneri; P. cedrella; P. corrugata; P. extremorientalis; Pseudomonas fluorescens; P. gessardii; P. libanensis; Pseudomonas mandelii; P. marginalis; P. migulae; P. mucidolens; P. orientalis; P. rhodesiae; P. synxantha; P. tolaasii;* and *P*. *veronii.*

The host cell can be selected from "Gram(-) Proteobacteria Subgroup 18." "Gram(-) Proteobacteria Subgroup 18" is defined as the group of all subspecies, varieties, strains, and other sub-special units of the species *P. fluorescens,* including those belonging, e.g., to the following (with the ATCC or other deposit numbers of exemplary strain(s) shown in parenthesis): *P. fluorescens* biotype A, also called biovar 1 or biovar I (ATCC 13525); *P*. *fluorescens* biotype B, also called biovar 2 or biovar II (ATCC 17816); *P. fluorescens* biotype C, also called biovar 3 or biovar III (ATCC 17400); *P. fluorescens* biotype F, also called biovar 4 or biovar IV (ATCC 12983); *P. fluorescens* biotype G, also called biovar 5 or biovar V (ATCC 17518); *P. fluorescens* biovar VI; *P. fluorescens* Pf0-1; *P. fluorescens* Pf-5 (ATCC BAA-477); *P. fluorescens* SBW25; and *P. fluorescens subsp. cellulosa* (NCIMB 10462).

The host cell can be selected from "Gram(-) Proteobacteria Subgroup 19." "Gram(-) Proteobacteria Subgroup 19" is defined as the group of all strains of *P*. *fluorescens* biotype A. A particularl strain of this biotype is *P. fluorescens* strain MB101 (see US Patent No. 5,169,760 to Wilcox), and derivatives thereof. An example of a derivative thereof is *P*. *fluorescens* strain MB214, constructed by inserting into the MB101 chromosomal asd (aspartate dehydrogenase gene) locus, a native *E. coli* PlacI-IacI-lacZYA construct (i.e. in which PlacZ was deleted).

Also disclosed herein is a host cell of any of the Proteobacteria of the order Pseudomonadales. In a particular embodiment, the host cell is any of the Proteobacteria of the family *Pseudomonadaceae.*

Additional *P. fluorescens* strains that can be used in the present invention include *P*. *fluorescens* Migula and *P. fluorescens* Loitokitok, having the following ATCC designations: (NCB 8286); NRRL B-1244; NCIB 8865 strain CO1; NCIB 8866 strain CO2; 1291 (ATCC 17458; IFO 15837; NCIB 8917; LA; NRRL B-1864; pyrrolidine; PW2 (ICMP 3966; NCPPB 967; NRRL B-899); 13475; NCTC 10038; NRRL B-1603 (6; IFO 15840); 52-1C; CCEB 488-A (BU 140); CCEB 553 OEM 15/47); IAM 1008 (AHH-27); LAM 1055 (AHH-23); 1 (IFO 15842); 12 (ATCC 25323; NIH 11; den Dooren de Jong 216); 18 (IFO 15833; WRRL P-7); 93 (TR-10); 108 (52-22; IFO 15832); 143 (IFO 15836; PL); 149 (2-40-40; IFO 15838); 182 (IFO 3081; PJ 73); 184 (IFO 15830); 185 (W2 L-1); 186 (IFO 15829; PJ 79); 187 (NCPPB 263); 188 (NCPPB 316); 189 (PJ227; 1208); 191 (IFO 15834; PJ 236; 22/1); 194 (Klinge R-60; PJ 253); 196 (PJ 288); 197 (PJ 290); 198 (PJ 302); 201 (PJ 368); 202 (PJ 372); 203 (PJ 376); 204 (IFO 15835; PJ 682); 205 (PJ 686); 206 (PJ 692); 207 (PJ 693); 208 (PJ 722); 212 (PJ 832); 215 (PJ 849); 216 (PJ 885); 267 (B-9); 271 (B-1612); 401 (C71A; IFO 15831; PJ 187); NRRL B-3178 (4; IFO 15841); KY 8521; 3081; 30-21; (IFO 3081); N; PYR; PW; D946-B83 (BU 2183; FERM-P 3328); P-2563 (FERM-P 2894; IFO 13658); IAM-1126 (43F); M-1; A506 (A5-06); A505 (A5-05-1); A526 (A5-26); B69; 72; NRRL B-4290; PMW6 (NCIB 11615); SC 12936; A1 (IFO 15839); F 1847 (CDC-EB); F 1848 (CDC 93); NCLB 10586; P17; F-12; AmMS 257; PRA25; 6133D02; 6519E01; Nl; SC15208; BNL-WVC; NCTC 2583 (NCIB 8194); H13; 1013 (ATCC 11251; CCEB 295); IFO 3903; 1062; or Pf-5.

### Fermentation

The term "fermentation" includes both embodiments in which literal fermentation is employed and embodiments in which other, non-fermentative culture modes are employed. Fermentation may be performed at any scale. In one embodiment, the fermentation medium may be selected from among rich media, minimal media, and mineral salts media; a rich medium may be used, but is typically avoided. In another embodiment either a minimal medium or a mineral salts medium is selected. In still another embodiment, a minimal medium is selected.

Mineral salts media consists of mineral salts and a carbon source such as, e.g., glucose, sucrose, or glycerol. Examples of mineral salts media include, e.g., M9 medium, *Pseudomonas* medium (ATCC 179), Davis and Mingioli medium (see, BD Davis & ES Mingioli (1950) J. Bact. 60:17-28). The mineral salts used to make mineral salts media include those selected from among, e.g., potassium phosphates, ammonium sulfate or chloride, magnesium sulfate or chloride, and trace minerals such as calcium chloride, borate, and sulfates of iron, copper, manganese, and zinc. Typically, no organic nitrogen source, such as peptone, tryptone, amino acids, or a yeast extract, is included in a mineral salts medium. Instead, an inorganic nitrogen source is used and this may be selected from among, e.g., ammonium salts, aqueous ammonia, and gaseous ammonia. A mineral salts medium will typically contain glucose as the carbon source. In comparison to mineral salts media, minimal media can also contain mineral salts and a carbon source, but can be supplemented with, e.g., low levels of amino acids, vitamins, peptones, or other ingredients, though these are added at very minimal levels.

In one embodiment, media can be prepared using the components listed in Table 7 below. The components can be added in the following order: first (NH₄)HPO4, KH₂PO₄ and citric acid can be dissolved in approximately 30 liters of distilled water; then a solution of trace elements can be added, followed by the addition of an antifoam agent, such as Ucolub N 115. Then, after heat sterilization (such as at approximately 121°C), sterile solutions of glucose MgSO₄ and thiamine-HCL can be added. Control of pH at approximately 6.8 can be achieved using aqueous ammonia. Sterile distilled water can then be added to adjust the initial volume to 371 minus the glycerol stock (123 mL). The chemicals are commercially available from various suppliers, such as Merck. This media can allow for a high cell density cultivation (HCDC) for growth of *Pseudomonas* species and related bacteria. The HCDC can start as a batch process which is followed by a two-phase fed-batch cultivation. After unlimited growth in the batch part, growth can be controlled at a reduced specific growth rate over a period of 3 doubling times in which the biomass concentration can increased several fold. Further details of such cultivation procedures is described by Riesenberg, D et al. (1991) "High cell density cultivation of Escherichia coli at controlled specific growth rate" J Biotechnol 20(1): 17-27.

**Table 7: Medium composition**

| | |
|---|---|
| Component | Initial concentration |
| KH₂PO₄ | 13.3 gl⁻¹ |
| (NH₄)₂HPO₄ | 4.0 g l⁻¹ |
| Citric acid | 1.7 g l⁻¹ |
| MgSO₄-7H₂O | 1.2g l⁻¹ |
| Trace metal solution | 10 ml l⁻¹ |
| Thiamin HCl | 4.5 mg l⁻¹ |
| Glucose-H₂O | 27.3 g l⁻¹ |
| Antifoam Ucolub N115 | 0.1 ml l⁻¹ |
| | |
| Feeding solution | |
| MgSO₄-7H₂O | 19.7 g l⁻¹ |
| Glucose-H₂O | 770 g l⁻¹ |
| NH₃ | 23 g |
| | |
| Trace metal solution | |
| Fe(ll1) citrate | 6 g l⁻¹ |
| MnCl₂-4H₂O | 1.5 g l⁻¹ |
| ZmCH₂COOl₂-2H₂O | 0.8 g l⁻¹ |
| H₃BO₃ | 0.3 g l⁻¹ |
| Na₂MoO₄-2H₂O | 0.25 g l⁻¹ |
| CoCl₂ 6H₂O | 0.25 g l⁻¹ |
| CuCl₂ 2H₂O | 0.15 g l⁻¹ |
| ethylene | 0.84 g l⁻¹ |
| dinitrilo-tetracetic acid Na₂-2H₂O | |
| (Tritriplex III, Merck) | |

The expression system according to the present invention can be cultured in any fermentation format. For example, batch, fed-batch, semi-continuous, and continuous fermentation modes may be employed herein.

The expression systems according to the present invention are useful for transgene expression at any scale (i.e. volume) of fermentation. Thus, e.g., microliter-scale, centiliter scale, and deciliter scale fermentation volumes may be used; and 1 Liter scale and larger fermentation volumes can be used. In one embodiment, the fermentation volume will be at or above 1 Liter. In another embodiment, the fermentation volume will be at or above 5 Liters, 10 Liters, 15 Liters, 20 Liters, 25 Liters, 50 Liters, 75 Liters, 100 Liters, 200 Liters, 500 Liters, 1,000 Liters, 2,000 Liters, 5,000 Liters, 10,000Liters or 50,000 Liters.

In the present invention, growth, culturing, and/or fermentation of the transformed host cells is performed within a temperature range permitting survival of the host cells, such as a temperature within the range of about 4°C to about 55°C, inclusive. In addition, "growth" is used to indicate both biological states of active cell division and/or enlargement, as well as biological states in which a non-dividing and/or non-enlarging cell is being metabolically sustained, the latter use being synonymous with the term "maintenance."

### Cell Density

An additional advantage in using *P. fluorescens* in expressing recombinant mammalian proteins includes the capacity of *P. fluorescens* to be grown in high cell densities compared to *E. coli* or other bacterial expression systems. To this end, *P. fluorescens* expressions systems according to the present invention can provide a cell density of about 20 g/L or more. The *P. fluorescens* expressions systems according to the present invention can likewise provide a cell density of at least about 70 g/L, as stated in terms of biomass per volume, the biomass being measured as dry cell weight.

In one embodiment, the cell density will be at least 20 g/L. In another embodiment, the cell density will be at least 25 g/L, 30 g/L, 35 g/L, 40 g/L, 45 g/L, 50 g/L, 60 g/L, 70 g/L, 80 g/L, 90 g/L., 100 g/L, 110 g/L, 120 g/L, 130 g/L, 140 g/L, or at least 150 g/L.

In another embodiments, the cell density at induction will be between 20 g/L and 150 g/L;, 20 g/L and 120 g/L; 20 g/L and 80 g/L; 25 g/L and 80 g/L; 30 g/L and 80 g/L; 35 g/L and 80 g/L; 40 g/L and 80 g/L; 45 g/L and 80 g/L; 50 g/L and 80 g/L; 50 g/L and 75 g/L; 50 g/L and 70 g/L; 40 g/L and 80 g/L.

### Isolation and Purification

The proteins of this invention may be isolated purified to substantial purity by standard techniques well known in the art, including including, but not limited to, ammonium sulfate or ethanol precipitation, acid extraction, anion or cation exchange chromatography, phosphocellulose chromatography, hydrophobic interaction chromatography, affinity chromatography, nickel chromatography, hydroxylapatite chromatography, reverse phase chromatography, lectin chromatography, preparative electrophoresis, detergent solubilization, selective precipitation with such substances as column chromatography, immunopurification methods, and others. For example, proteins having established molecular adhesion properties can be reversibly fused a ligand.. With the appropriate ligand, the protein can be selectively adsorbed to a purification column and then freed from the column in a relatively pure form. The fused protein is then removed by enzymatic activity. In addition, protein can be purified using immunoaffinity columns or Ni-NTA columns. General techniques are further described in, for example, R. Scopes (1982) Protein Purification: Principles and Practice, Springer-Verlag: N.Y.; Deutscher (1990) Guide to Protein Purification, Academic Press; U.S. Patent No. 4,511,503; S. Roe (2001) Protein Purification Techniques: A Practical Approach, Oxford Press; D. Bollag, et al. (1996) Protein Methods, Wiley-Lisa, Inc.; AK Patra et al. (2000) Protein Expr Purif, 18(2):182-92; and R. Mukhija, et al. (1995) Gene 165(2):303-6. See also, for example, Deutscher (1990) "Guide to Protein Purification," Methods in Enzymology vol. 182, and other volumes in this series; Coligan, et al. (1996 and periodic Supplements) Current Protocols in Protein Science, Wiley/Greene, NY; and manufacturer's literature on use of protein purification products, e.g., Pharmacia, Piscataway, N.J., or Bio-Rad, Richmond, Calif. Combination with recombinant techniques allow fusion to appropriate segments, e.g., to a FLAG sequence or an equivalent which can be fused via a protease-removable sequence. See also, for example: Hochuli (1989) Chemische Industrie 12:69-70; Hochuli (1990) "Purification of Recombinant Proteins with Metal Chelate Absorbent" in Setlow (ed.) Genetic Engineering, Principle and Methods 12:87-98, Plenum Press, NY; and Crowe, et al. (1992) QIAexpress: The High Level Expression & Protein Purification System QUIAGEN, Inc., Chatsworth, Calif.

Detection of the expressed protein is achieved by methods known in the art and include, for example, radioimmunoassays, Western blotting techniques or immunoprecipitation.

The recombinantly produced and expressed enzyme can be recovered and purified from the recombinant cell cultures by numerous methods, for example, high performance liquid chromatography (HPLC) can be employed for final purification steps, as necessary.

Certain proteins expressed in this invention may form insoluble aggregates ("inclusion bodies"). Several protocols are suitable for purification of proteins from. inclusion bodies. For example, purification of inclusion bodies typically involves the extraction, separation and/or purification of inclusion bodies by disruption of the host cells, e.g., by incubation in a buffer of 50 mM TRIS/HCl pH 7.5, 50 mM NaCl, 5 mM MgCl₂, 1 mM DTT, 0.1 mM ATP, and 1 mM PMSF. The cell suspension is typically lysed using 2-3 passages through a French Press. The cell suspension can also be homogenized using a Polytron (Brinkman Instruments) or sonicated on ice. Alternate methods of lysing bacteria are apparent to those of skill in the art (see, e.g., Sambrook, J., E. F. Fritsch and T. Maniatis eds. (1989) "Molecular Cloning: A Laboratory Manual", 2d ed., Cold Spring Harbor Laboratory Press; Ausubel et al., eds. (1994) Current Protocols in Molecular Biology*).*

If necessary, the inclusion bodies can be solubilized, and the lysed cell suspension typically can be centrifuged to remove unwanted insoluble matter. Proteins that formed the inclusion bodies may be renatured by dilution or dialysis with a compatible buffer. Suitable solvents include, but are not limited to urea (from about 4 M to about 8 M), formamide (at least about 80%, volume/volume basis), and guanidine hydrochloride (from about 4 M to about 8 M). Although guanidine hydrochloride and similar agents are denaturants, this denaturation is not irreversible and renaturation may occur upon removal (by dialysis, for example) or dilution of the denaturant, allowing re-formation of immunologically and/or biologically active protein. Other suitable buffers are known to those skilled in the art.

Alternatively, it is possible to purify the recombinant proteins from the host periplasm. After lysis of the host cell, when the recombinant protein is exported into the periplasm of the host cell, the periplasmic fraction of the bacteria can be isolated by cold osmotic shock in addition to other methods known to those skilled in the art. To isolate recombinant proteins from the periplasm, for example, the bacterial cells can be centrifuged to form a pellet. The pellet can be resuspended in a buffer containing 20% sucrose. To lyse the cells, the bacteria can be centrifuged and the pellet can be resuspended in ice-cold 5 mM MgSO₄ and kept in an ice bath for approximately 10 minutes. The cell suspension can be centrifuged and the supernatant decanted and saved. The recombinant proteins present in the supernatant can be separated from the host proteins by standard separation techniques well known to those of skill in the art.

An initial salt fractionation can separate many of the unwanted host cell proteins (or proteins derived from the cell culture media) from the recombinant protein of interest. One such example can be ammonium sulfate. Ammonium sulfate precipitates proteins by effectively reducing the amount of water in the protein mixture. Proteins then precipitate on the basis of their solubility. The more hydrophobic a protein is, the more likely it is to precipitate at lower ammonium sulfate concentrations. A typical protocol includes adding saturated ammonium sulfate to a protein solution so that the resultant ammonium sulfate concentration is between 20-30%. This concentration will precipitate the most hydrophobic of proteins. The precipitate is then discarded (unless the protein of interest is hydrophobic) and ammonium sulfate is added to the supernatant to a concentration known to precipitate the protein of interest. The precipitate is then solubilized in buffer and the excess salt removed if necessary, either through dialysis or diafiltration. Other methods that rely on solubility of proteins, such as cold ethanol precipitation, are well known to those of skill in the art and can be used to fractionate complex protein mixtures.

The molecular weight of a recombinant protein can be used to isolated it from proteins of greater and lesser size using ultrafiltration through membranes of different pore size (for example, Amicon or Millipore membranes). As a first step, the protein mixture can be ultrafiltered through a membrane with a pore size that has a lower molecular weight cut-off than the molecular weight of the protein of interest. The retentate of the ultrafiltration can then be ultrafiltered against a membrane with a molecular cut off greater than the molecular weight of the protein of interest. The recombinant protein will pass through the membrane into the filtrate. The filtrate can then be chromatographed as described below.

Recombinant protiens can also be separated from other proteins on the basis of its size, net surface charge, hydrophobicity, and affinity for ligands. In addition, antibodies raised against proteins can be conjugated to column matrices and the proteins immunopurified. All of these methods are well known in the art. It will be apparent to one of skill that chromatographic techniques can be performed at any scale and using equipment from many different manufacturers (e.g., Pharmacia Biotech).

### Renaturation and Refolding

Insoluble protein can be renatured or refolded to generate secondary and tieriary protein structure conformation. Protein refolding steps can be used, as necessary, in completing configuration of the recombinant product. Refolding and renaturation can be accomplished using an agent that is known in the art to promote dissociation/association of proteins. For example, the proteincan be incubated with dithiothreitol followed by incubation with oxidized glutathione disodium salt followed by incubation with a buffer containing a refolding agent such as urea.

Recombinant protein can also be renatured, for example, by dialyzing it against phosphate-buffered saline (PBS) or 50 mM Na-acetate, pH 6 buffer plus 200 mM NaCl. Alternatively, the protein can be refolded while immobilized on a column, such as the Ni-NTA column by using a linear 6M-1M urea gradient in 500 mM NaCl, 20% glycerol, 20 mM Tris/HCl pH 7.4, containing protease inhibitors. The renaturation can be performed over a period of 1.5 hours or more. After renaturation the proteins can be eluted by the addition of 250 mM immidazole. Immidazole can be removed by a final dialyzing step against PBS or 50 mM sodium acetate pH 6 buffer plus 200 mM NaCl. The purified protein can be stored at 4°C or frozen at -80°C.

Other methods include, for example, those that may be described in: MH Lee et al. (2002) Protein Expr. Purif. 25(1):166-73; W.K. Cho et al. (2000) J. Biotechnology 77(2-3):169-78; Deutscher (1990) "Guide to Protein Purification," Methods in Enzymology vol. 182, and other volumes in this series; Coligan, et al. (1996 and periodic Supplements) Current Protocols in Protein Science, Wiley/Greene, NY; S. Roe (2001) Protein Purification Techniques: A Practical Approach, Oxford Press; D. Bollag, et al. (1996) Protein Methods, Wiley-Lisa, Inc.

### A ctive Protein or Peptide Analysis

Typically, an "active" protein includes proteins that have a biological function or biological effect comparable to the corresponding native protein. In the context of proteins this typically means that a polynucleotide or polypeptide comprises a biological function or effect that has at least about 20%, about 50%, about 60%, about 70%, about 75%, about 80%, about 85%, about 90%, about 95%, about 98% or 100% biological function compared to the corresponding native protein using standard parameters. The determination of protein activity can be performed utilizing corresponding standard, targeted comparative biological assays for particular proteins. One indication that a recombinant protein biological function or effect is that the recombinant polypeptide is immnunologically cross reactive with the native polypeptide.

Active proteins typically have a specific activity of at least 20%, 30%, 40%, 50%, 60%, 70%, 80%, 90%, or 95% that of the native mammalian protein. Further, the substrate specificity (k_{cat}/Kₘ) is optionally substantially similar to the native mammalian protein. Typically, k_{cat}/Kₘ will be at least 30%, 40%, 50%, 60%, 70%, 80%, or 90% that of the native protein. Methods of assaying and quantifying measures of protein and peptide activity and substrate specificity (k_{cat}/Kₘ), are well known to those of skill in the art.

The activity of a recombinant mammalian protein can be measured by any protein specific conventional or standard *in vitro* or *in vivo* assay known in the art. The activity of the *Pseudonmonas* produced recombinant mammalian protein can be compared with the activity of the corresponding native mammalian protein to determine whether the recombinant mammalian protein exhibits substantially similar or equivalent activity to the activity generally observed in the native protein under the same or similar physiological conditions.

The activity of the recombinant protein can be compared with a previously established native protein standard activity. Alternatively, the activity of the recombinant protein can be determined in a simultaneous, or sunstantially simultaneous, comparative assay with the native protein. For example, an *in vitro* assays can be used to determine any detectable interaction between a recombinant protein and a target, e.g. between an expressed enzyme and substrate, between expressed hormone and hormone receptor, between expressed antibody and antigen, etc. Such detection can include the measurement of colorimetric changes, proliferation changes, cell death, cell repelling, changes in radioactivity, changes in solubility, changes in molecular weight as measured by gel electrophoresis and/or gel exclusion methods,phosphorylation abilities, antibody specificity assays such as ELISA assays, etc. In addition, *in vivo* assays include, but are not limited to, assays to detect physiological effects of the *Pseudomonas* produced protein in comparison to physiological effects of the native protein, e.g. weight gain, change in electrolyte balance, change in blood clotting time, changes in clot dissolution and the induction of antigenic response. Generally, any *in vitro* or *in vivo* assay can be used to determine the active nature of the *Pseudomonas* produced recombinant mammalian protein that allows for a comparative analysis to the native protein so long as such activity is assayable. Alternatively, the proteins produced in the present invention can be assayed for the ability to stimulate or inhibit interaction between the protein and a molecule that normally interacts with the protein, e.g. a substrate or a component of the signal pathway that the native protein normally interacts. Such assays can typically include the steps of combining the protein with a substrate molecule under conditions that allow the protein to interact with the target molecule, and detect the biochemical consequence of the interaction with the protein and the target molecule.

Assays that can be utilized to determine protein activity are described, for example, in: Ralph, P. J., et al. (1984) J. Immunol. 132:1858; Saiki et al. (1981) J. Immunol. 127:1044; Steward, W. E. II (1980) The Interferon Systems. Springer-Verlag, Vienna and New York; Broxmeyer, H. E., et al. (1982) Blood 60:595; Sambrook, J., E. F. Fritsch and T. Maniatis eds. (1989) "Molecular Cloning: A Laboratory Manual", 2d ed., Cold Spring Harbor Laboratory Press; Berger, S. L. and A. R. Kimmel eds. (1987) "Methods in Enzymology: Guide to Molecular Cloning Techniques", Academic Press; AK Patra et al. (2000) Protein Expr Purif 18(2):182-92; Kodama et al. (1986) J. Biochem. 99:1465-1472; Stewart et al. (1993) Proc. Nat'l Acad. Sci. USA 90:5209-5213; Lombillo et al. (1995) J. Cell Biol. 128:107-115; Vale et al. (1985) Cell 42:39-50.

### EXAMPLES

### Bacterial Strains and Growth Conditions.

Unless otherwise specified, all strains used for all *Pseudomonas* expression testing were based on *P. fluorescens* strain MB101. *E. coli* strains JM109 (Promega), XL2 Blue (Stratagene) or Top 10 (Invitrogen) were used for general cloning. For *E. coli* expression studies, BL21(DE3) Gold was used. *P. fluorescens* strains were grown in either LB or minimal salts medium supplemented with 15 ug/mL tetracycline and 30 ug/mL kanamycin as needed at 30°C. *E. coli* strains were grown in LB supplemented with 30 ug/mL kanamycin and/or 15 ug/mL chloramphenicol, or 15 ug/mL tetracycline as needed at 37°C. Cells were induced with 0.3mM IPTG following growth phase.

### Protein Activity Detection (ELISA Assay)

Plates were coated by adding 200 µL of the β-galactosidase solution at 10 µg/mL in PBS (pH 7.6) to each well of the microtiter plate. Plates were incubated at room temperature for 16 hrs, then washed 3 times with 200 µL PBS + 0.1% Tween-20 (PBS-T). Primary antibody was diluted in PBS with 2% nonfat dry milk (w/v). 200 µL of the diluted antibody was added to each well and incubated at room temperature for 1 hr. The plates were then washed 4 times with 200 µL PBS-T. The secondary antibody was also diluted in PBS with 2% non fat dry milk (w/v) and to each well, 200 µL was added and incubated at room temperature for 1.5-2 hours. The plates were then washed 4 times with PBS-T. A tertiary antibody is used to detect the scFv antibodies: alkaline phosphatase conjugated sheep antimouse antibody (Sigma-Aldrich, St. Louis, MO, USA cat #A5324). To each desired well was added 200 µL of diluted antibody solution (or PBS-T) and incubated at room temperature for 1.5 hours. The plates were then washed 4 times with PBS-T. To each well was added 200 µl of the freshly prepared Sigma Fast pNPP substrate (Sigma catalogue #R-2770). After 30 minutes, the reaction was stopped by adding 50 µL 3M NaOH to each well and absorbance was read at 405 nm.

### Fermentation

The inoculum for the fermentor culture for *P. fluorescens* is generated by inoculating a shake flask containing 600 mL of a chemically defined medium supplemented with yeast extract and dextrose. Tetracycline is typically added to ensure maintenance of the recombinant plasmid in the starter culture during its overnight incubation as well as in the fermentor. The shake flask culture is then aseptically transferred to a 20L fermentor containing a chemically defined medium designed to support a high biomass, without yeast extract supplementation. Oxygen is maintained at a positive level in the liquid culture by regulating the air flow into the fermentor and the agitator mixing rate; the pH is maintained at greater than 6.0 through the addition of aqueous ammonia. The fed-batch high density fermentation process is divided into an initial growth phase of approximately 24h and gene expression (induction) phase in which an inducer is added to initiate recombinant gene expression. Glucose, in the form of corn syrup, is fed throughout the fermentation process at limiting concentrations. The target cell density for initiating the induction phase is typically 150 OD units at 575nm. The induction phase of the fermentation is typically allowed to go for approximately 45 to 55 hours. During this phase, samples are withdrawn from the fermentor for various analyses to determine the level of target gene expression, cell density, etc.

For each fermentation experiment for *E. coli,* a frozen glycerol stock is removed from -80°C storage, thawed and diluted before inoculating a shake flask containing 600 mL of LB broth supplemented with kanamycin. The shake flask culture is incubated at 37°C with shaking at 300 rpm overnight and then aseptically transferred to a 20L fermentor containing complex medium. Temperature in the fermentor is maintained at 37°C, pH at 7 through the addition of aqueous ammonia and phosphoric acid, and dissolved oxygen at greater than 20%. After a brief initial batch phase, glycerol is fed at rates increased stepwise to maintain excess carbon. When the cell density reaches 24-28 OD units at 600nm, recombinant expression is effected by addition of an inducer, such as isopropyl-thiogalactoside (IPTG). The induction phase of the fermentation typically continues for approximately 3 to 5 hours as the fermentor reached volumetric capacity or as the growth rate began to decrease significantly. During this phase, samples are withdrawn from the fermentor for various analyses to determine the level of target gene expression, cell density, etc.

### Cell fractionation and SDS-PAGE analysis.

Samples are normalized to A575=30, and 1 mL normalized culture is pelleted. Cells are resuspended in 1mL lysis buffer (50 mM Tris base; 200mM NaCl; 5% v/v glycerol; 20 mM EDTA disodium salt; 0.5% v/v Triton X-100; 1 mM DTT). A protease inhibitor cocktail specific for bacterial lysates (Sigma#P8465) is added to a 1X concentration. The resuspended cells are added to a 2 ml screw cap microfuge tube approximately ¾ full with 0.1 mm glass beads and the cells are mechanically lysed using 4, 1 minute incubations in a BioSpec bead mill at the highest setting. Cells are kept on ice between incubations. Approximately 100uL of lysed cell solution is removed from beads, transferred into a new tube and pelleted. The supernatant (soluble fraction) is removed to a new tube. The pellet (insoluble fraction) is resuspended in an equal volume (100 uL) of lysis buffer plus protease inhibitor. Five uL of each sample is added to 5uL of 2X LDS loading buffer (Invitrogen) and loaded onto a 4-12% or 10% Bis-Tris NuPAGE gel (Invitrogen) and run in either 1X MES or IX MOPS buffer as indicated.

### Example 1: Expression of scFV in the Cytoplasm

Single chain antibody fragments (scFV) are finding increased use as diagnostic and therapeutic agents. These relatively small proteins are made by fusing together genes coding the variable light and heavy chains of an immunoglobulin.

### Cloning of Gall3 scFv

The Gall3 scFv gene (Genbank accession number AF238290), cloned into the phage display vector pCANTAB6, (see P Martineau et al. (1998) J. Mol. Biol. 280(1):117-27) was used as template to amplify a 774 base pair product, which was subsequently cloned into the pCR2.1 TOPO vector (Invitrogen, Carlsbad, CA, USA). The scFv gene was excised from the TOPO vector with SpeI and SalI restriction enzymes (New England Biolabs, Beverly, MA, USA) and cloned into the SpeI and XhoI sites of the *P. fluorescens* vector pMYC1803, downstream of the Ptac promoter, to produce pDOW1117. The resulting plasmids were electroporated into *P. fluorescens.* The Gall3 gene was cloned into the pET24d+ expression vector (Novagen, Madison, WI, USA), following amplification such that SalI and NcoI sites flanked the coding sequence. The PCR products were digested with SalI and NcoI and cloned into the same sites of pET24d+ vector downstream of the T7 promoter. The newly formed construct was then used to transform XL2 Blue competent cells. Once sequence was confirmed, the DNA construct was used to transform BL21(DE3) Gold (Stratagene, San Diego, CA, USA) for expression.

### Expression of a Single Chain Antibody Fragment (scFv) in E. coli and P. fluorescens

scFv molecules were expressed in both *E. coli* and *P. fluorescens,* among them an scFv with binding activity to the *E. coli* protein β-galactosidase single chain antibody ga113 (P. Martineau et al., "Expression of an antibody fragment at high levels in the bacterial cytoplasm, " J. Mol. Biol. 280(1):117-27 (1998)). *P. fluorescens* expressed about six-fold more protein than *E. coli* during 20L fermentation, with 3.1 g/L yield in *P. fluorescens* and 0.5 g/L yield in *E. coli* as determined by SDS-PAGE and densitometry (see Table 8). P. *fluorescens* expressed about 96% soluble protein, whereas *E. coli* expresses only 48% soluble protein.

**Table 8: Gal13 fermentation summary (*compared to BSA standards)**

| | ***E. coli*** | ***P. fluorescens*** | ***Pf*/*Ec*** |
|---|---|---|---|
| **Fermentatino Time (hr)** | 8-9 | 70 | 8 |
| **Max hGH titre (*g/L)** | 0.4 (85%cv) | 3.1 (24%cv) | 8 |
| **Dry biomass (g/L)** | ND (30) | 59 | (2) |
| **hGH/biomass (%w/w)** | (1) | 5 | (5) |

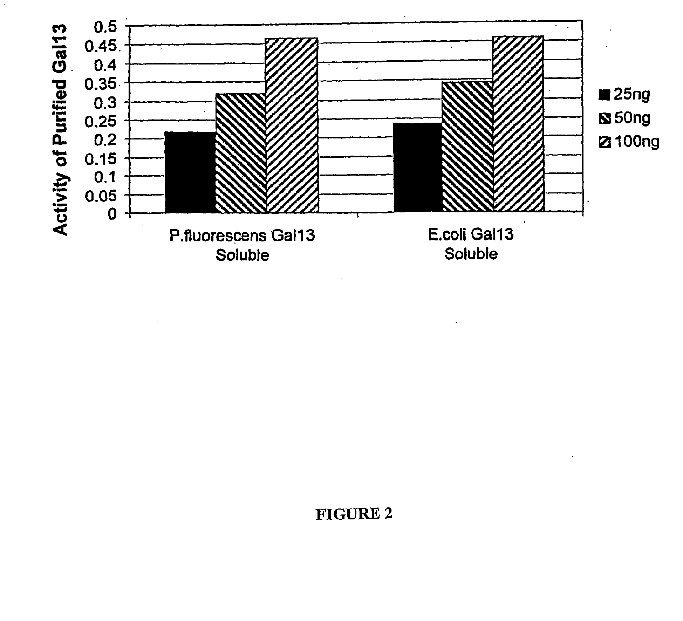

Material purified from both expression systems was found to be active in an enzyme-linked immunosorbant assay (ELISA) as shown in Figure 2. Material was also purified from the soluble fraction only of equal lysate volumes from lysates of both strains using affinity chromatography. Finally, the overall volumetric recovery for the *P. fluorescens* process is approximately 20 fold more efficient than for *E. coli,* 1.34 g/L vs. 0.07 g/L.

### Example 2: Expression of human γ-IFN in the Cytoplasm

### Cloning of Human gamma-Interferon

Human gamma interferon (hu-γIFN, Genbank accession X13274) was amplified from a human spleen cDNA library (Invitrogen, Carlsbad, CA, USA; catalogue #10425-015) such that it lacked the native secretion signal, with the N-terminus of the recombinant γ-IFN beginning as Met-Cys-Tyr-Cys-Gln-Asp-Pro as described in PW Gray et al. (1982) Nature 298:859-63. The resulting product was cloned into the pCR2.1 TOPO vector and the sequence was confirmed. The hu-γIFN gene was excised from the TOPO vector with SpeI and XhoI restriction enzymes and cloned into the same sites of pMYC1803. In a separate reaction, hu-γIFN was amplified such that AflIII and XhoI sites flanked that coding sequence. The resulting fragment was cloned into the TOPO-TA vector (Invitrogen) and transformed into chemically competent *E. coli* JM109 cells (Promega, Madison, WI, USA). The gene was isolated by digesting with AflIII and XhoI (New England Biolabs), cloned into the NcoI and XhoI sites of pET24d+ (Novagen, Madison, WI, USA) downstream of the T7 promoter, and transformed into JM109. A positive clone was transformed into *E. coli* BL21(DE3) cells (Novagen) to test for expression.

### Human gamma-Interferon Purification

Frozen cell paste from *P. fluorescens* cultures was thawed and re-suspended in lysis buffer (50 mM potassium phosphate, pH 7.2 containing 50 mM NaCl, 10 mM EDTA (ethylenediaminetetraacetic acid, catalog number BPII8-500, Fisher Scientific, Springfield, NJ, USA), 1 mM PMSF (phenylmethylsulfonyl fluoride, catalog number P-7626, Sigma, St. Louis, MO), 1mM dithiothreitol (catalog number D-0632, Sigma), and 1 mM benzamidine (catalog number B-6506, Sigma)) at a ratio of about 1 gram cell paste per 2 mL lysis buffer. Cells were broken by three passages through a microfluidizer (model 110Y, Microfluidics Corporation, Newton, MA, USA). Cell debris and unbroken cells were removed by centrifugation (for 60 min at 23,708 x g and 4°C using a Beckman Coulter centrifuge; model JA 25.50, Beckman Coulter, Inc., Fullerton, CA, USA). The resulting supernatant (cell-free extracts) was clarified by adding 10% w/v diatomaceous earth (Celite product, World Minerals, Inc., Goleta, CA, USA) and passing the result through a paper filter (Whatman 1, catalog number 1001-150, Whatman Paper Ltd., Maidstone, Kent, UK)) with vacuum filtration.

Clarified cell extracts were applied to a 3.2 cm x 13.5 cm chromatography column of SP-Sepharose FAST FLOW (6% cross-linked agarose bead material; catalog number 17-0709-10, Amersham Biosciences, Piscataway, NJ, USA) equilibrated in buffer A, at a flow rate of 0.5 mL/min. The composition of Buffer A was: 50mM HEPES, pH 7.8 (i.e. N-(2-hydroxyethyl)piperazine)N'-(2-ethanesulfonic acid), from Fisher Scientific, catalog number BP-310-100), 50mM NaCl, 1mM EDTA, and 0.02% sodium azide (catalog number 71289, Sigma Chemical Co.). After loading, the column was washed with 3 column volumes (column volume = 108 mL) buffer A and 5 column volumes of buffer A containing 0.4M NaCl. The column was further developed by applying a gradient of 0.4 M to 1 M NaCl in the same buffer at a flow rate of 2 mL/min for a total of 7 column volumes. Fractions containing pure IFN-γ were then pooled and dialyzed against 1X PBS (phosphate-buffered saline, pH 7.2) at 4°C. Protein was concentrated by ultrafiltration (using a YM30 ultrafiltration membrane; catalog no. 13722, from Millipore, Bedford, MA USA), then frozen in liquid nitrogen and stored at 80°C.

### Expression of Human γ-Interferon in E. coli and P. fluorescens

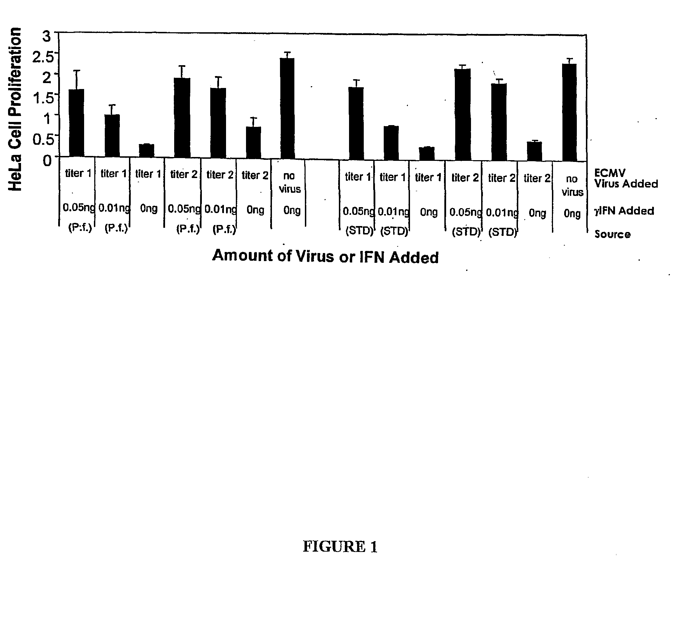

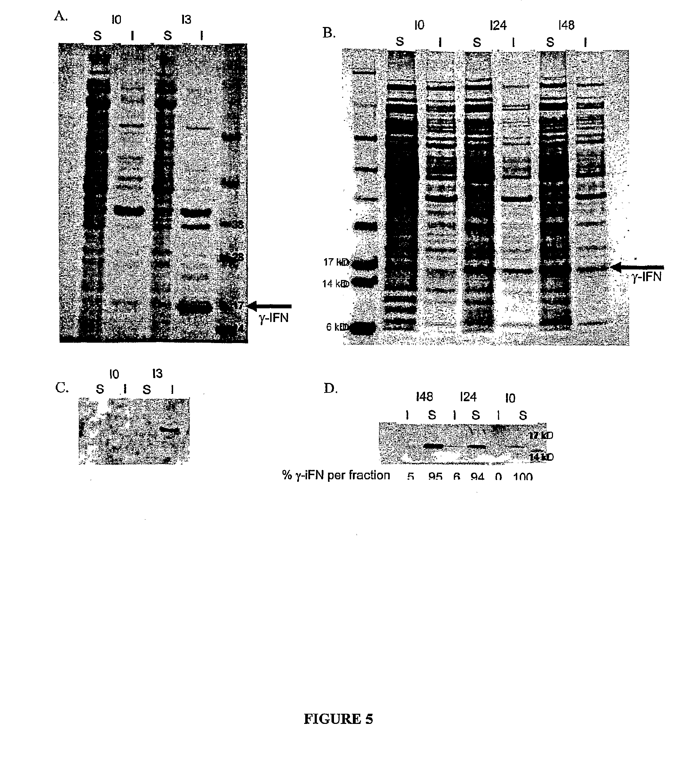

Human γ-interferon is produced commercially by fermentation of *E. coli* expressing the γ-IFN gene. The protein is expressed cytoplasmically in an insoluble and inactive form. In order to produce the recombinant polypeptide as an active pharmaceutical ingredient, the interferon must be recovered, solubilized, refolded, and then purified. All these unit operations add greatly to the cost of goods (COGs) for this protein. A human spleen cDNA library was used as a template to amplify the γIFN cDNA without the native signal sequence and clone into *E. coli* and *P. fluorescens* expression vectors. *P. fluorescens* construct produced ∼4g/L of γIFN protein during a typical 20L fermentation reaction. SDS-PAGE and Western analyses of soluble and insoluble fractions show that the majority of the protein (95%) is present in the soluble fraction. Figure 1 shows that hu-γ-IFN purified from the soluble fraction of *P. fluorescens* samples displays activity comparable to a commercially available standard. Figure 5 and Table 9 show a comparison of expression of γ-IFN between *E. coli* and *P. fluorescens* expression systems.

**Table 9: γ-IFN fermentation summary (*compared to BSA standards)**

| | ***E. coli*** | ***P. fluorescens*** | ***Pf*/*Ec*** |
|---|---|---|---|
| **Fermentatino Time (hr)** | 7-9 | 55 | 6 |
| **Max hGH titre (*g/L)** | 3.9 | 4.5 | 1.5 |
| **Dry biomass (g/L)** | ∼22 | 100 | 4.5 |
| **hGH/biomass (%w/w)** | ∼17.7 | 4.5 | 0.25 |

### Assay of Human gamma Interferon Activity

Cell lines and media: Hela cells (catalogue no. CCL-2) and encephalomyocarditis virus (ECMV, catalogue no. VR-129B) were obtained from the American Type Culture Collection (Manassas, VA). HeLa cells were maintained in Eagles Modified Essential Medium (Cellgro EMEM, Mediatech, Herdon, VA, USA) with 10% fetal bovine serum (Gibco, Invitrogen, Carlsbad, CA, USA) at 37°C/ 5% CO₂.

The activity of purified hu-γIFN was assayed using a viral inhibition assay as previously described (JA Lewis (1987) in Lymphokines and Interferons: A Practical Approach MJ Clemens et al. (eds.) (IRL Press Ltd, Oxford, England). Briefly, HeLa cells were seeded in a 96-well microtiter plate at 3 X 10⁴ per well. After 24 hours, purified hu-γIFN isolated from *P. fluorescens,* or *E. coli* recombinant hu-γIFN (from R&D Systems, Minneapolis, MN, USA), was added to triplicate wells at 0, 0.01 or 0.05 ng per well. After preincubating the cells with hu-γIFN for 24 hours, ECMV was added at varying dilutions to sets of triplicate wells. The cells were incubated for 5 days, after which cell viability was measured using a cell proliferation ELISA that monitors 5-bromo-2'-deoxyuridine incorporation (catalogue no. 1647229, Roche Molecular Biochemicals, Indianapolis, IN, USA). Results are expressed as absorbance units, with greater absorbance resulting from the presences of a greater number of actively dividing (live) cells.

### Example 3: Expression of hGH in the Cytoplasm

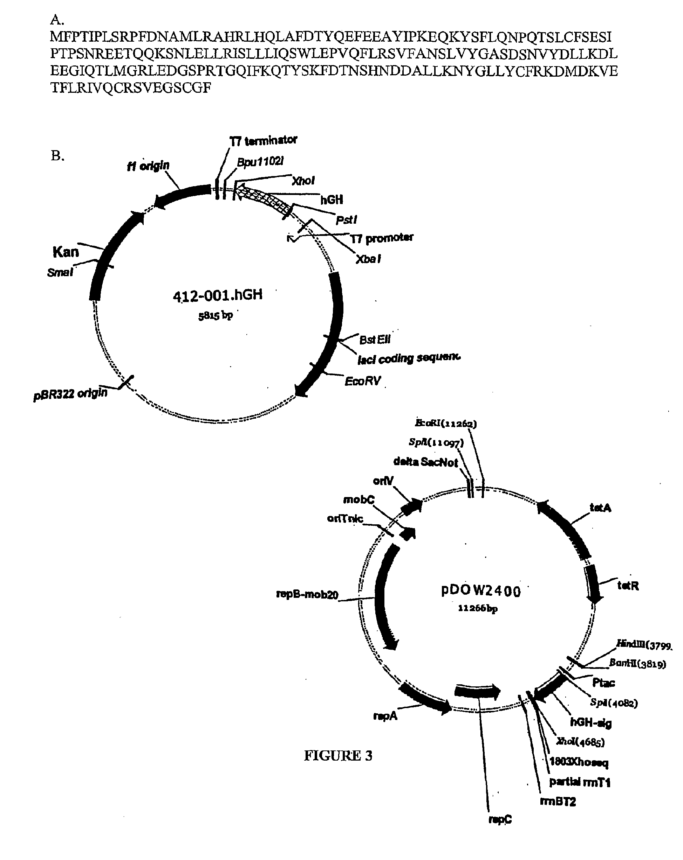

Primers were designed to amplify human growth hormone (hGH) from human cDNA libraries. For this study, hGH was amplified using AmpliTaq polymerase (Perkin Elmer) according to the manufacturer's protocol, using the above plasmid as template and primers ELVIrev and hgh-sig, with a PCR cycling profile of 95°C 2 minutes (95°C 60 seconds 42°C 120 seconds 72°C 3 minutes) 25X. The resulting product was purified using Wizard PCR DNA purification kit (Promega), digested with SpeI and XhoI restriction enzymes (New England Biolabs) and cloned into the same sites of pMYC1803 (see Figure 3). A mutation found in the amplified hGH was corrected by using the hgh-sigcorr primer with ELVIrev and repeating the PCR and cloning procedures.

**Primers used to clone hGH.**

| | |
|---|---|
| hGH-sig | |
| HGH-sigcorr | |
| ELVIfor | |
| ELVIrev | AGAGACTCGAGTCATTAGAAGCCACAGCTGCCCTCCAC |

### Purification of hGH

Following 20L fermentation, hGH was purified from the insoluble fraction of *E. coli* and *P. fluorescens* cells, with the exception that during DEAE FF elution a gradient from 0 to 0.5M NaCl was used in place of a 0.25M NaCl step.

### Expression of human growth hormone in E. coli vs. P. fluorescens.

The cDNA encoding human growth hormone was amplified from a human pituitary cDNA library. The native secretion signal sequence was removed, and an N-terminal methionine was engineered into the constructs for microbial expression. For *E. coli* expression, the pET25 vector containing the hGH gene was transformed into BL21(DE3), which contains an integrated T7 polymerase gene necessary for hGH transcription. *P*. *fluorescens* expression studies were carried out in the MB214 strain, which contains an integrated lacI gene to control expression from the Ptac promoter. Both expression systems were evaluated at the 20L fermentation scale. As shown in Table 10, *P. fluorescens* (Pf) outperformed *E. coli* (EC) in the amount of protein produced per gram of dry biomass (1.6X as much).

**Table 10: hGH fermentation summary (*compared to BSA standards)**

| | ***E. coli*** | ***P. fluorescens*** | ***Pf*/*Ec*** |
|---|---|---|---|
| **Fermentatino Time (hr)** | 7-9 | 55 | 6 |
| **Max hGH titre (*g/L)** | 2.6 (23%cv) | 7.3 (5%cv) | 3 |
| **Dry biomass (g/L)** | 37 | 66 | 2 |
| **hGH/biomass (%w/w)** | 7 | 11 | 1.6 |

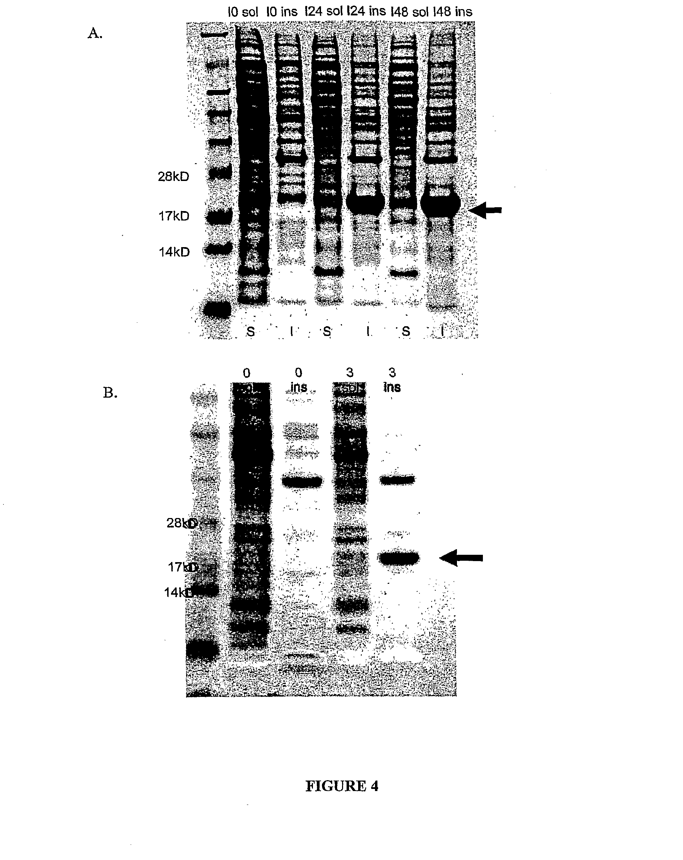

Cell fractionation and SDS-PAGE analysis show that hGH is found in the insoluble fraction in both expression systems (Figure 4). Surprisingly, approximately 7X more hGH monomer was purified from *P. fluorescens,* compared to *E. coli,* despite a difference of only 1.6X in protein production per gram of dry biomass.

**Table 11:CComparison of hGHpurification from E. coli and P. fluorescens**

| ***1.62-1.75 L portion of P. fluorescens containing rh-GH*** | | | |
|---|---|---|---|
| | **Wet Biomass (wet g)** | **Purified Monomer (mg)** | **Purified Dimer (mg)** |
| **Soluble** | minimal | NA | NA |
| **Lipid Extract** | None detected | -- | -- |
| **Lipid Insoluble** | 62.2-63.1 | 1483 | 346 |
| | | | |

| ***1.5-1.6 L portion of E. coli containing rh-GH*** | | | |
|---|---|---|---|
| **Soluble** | minimal | NA | NA |
| **Lipid Extract** | None detected | -- | -- |
| **Lipid Insoluble** | 35.0 | 200 | 333 |

### Example 4: Expression of Proteins in the Periplasm

### Characterization of Secretion Signal Peptides

*Pseudomonas fluorescens* secretion signal peptides were discovered by formation and expression of alkaline phosphatase (phoA) coding sequence-genomic DNA fusions and are described in more detail in U.S. Application No. 10/996,007, filed November 22, 2004. Six of the expressed fusions were further characterized as follows.

The cleavage site for the signal sequences for the secreted genes identified as phoA fusions was deduced by comparison to homolgous proteins from other Pseudomonads, by the SPScan program (Menne et al, 2000). The cleavage site of the putative lipoprotein was deduced by comparison to signal peptidase II motifs; signal peptidase II specifically cleaves the signal sequences of lipoproteins. All six of the signal peptides were analyzed using SignalP (a software program for analysis of putative signal peptides; available from the Center for Biological Sequence Analysis of the Technical University of Denmark, at http://www.cbs.dtu.dklservices/SignalP/.) Also see, Nielson et al. (1997) Protein Engineering 10:1-6. In some cases, a supplementary source was used to further characterize the identity of the signal peptide. This information is present in Table 12.

**Table 12. Identities of Secretion Signal Peptides**

| Identity | Putative Amino Acid Sequence |
|---|---|
| Putative porin El precursor, OprE | |
| Putative phosphate binding protein | Lys Leu Lys Arg Leu Met Ala Ala Met Thr Phe Val Ala Ala Gly |
| Putative azurin | |
| Putative periplasmic lipoprotein B precursor | Ile Lys Arg Asn Leu Leu Val Met Gly Leu Ala Val Leu Leu Ser |
| Putative Lys-Arg-Orn binding protein | |
| Putative Fe(III) binding protein | |

### Western analysis of the phoA fusion proteins to detect fusion proteins

To analyze whether the fusion proteins were produced, Western analysis with antibody to alkaline phosphatase was carried out on cultures separated by centrifugation into a whole-cell fraction (cytoplasm and periplasm) and a cell-free broth fraction. Of five strains for which the site of insertion was determined, four (putative azurin, putative phosphate binding protein, putative periplasmic lipoprotein B, putative Fe(III) binding protein) produced a fusion protein of the expected size, and one (putative oprE protein) produced a protein about 40 kD smaller than predicted, and one (putative Lys-Arg-Orn binding protein) produced a protein about 20 kD smaller than predicted.

Proteins were separated by SDS-PAGE and were transferred to nitrocellulose membrane at 40 V for one hour using the Xcell SureLockTM Mini-Cell and XCell II TM Blot Module (Invitrogen). Western experiments were performed using the instruction provided from SuperSignal West HisProbeTM Kit (Pierce).

### Construction, Expression, and Characterization of a pbp-hGH Fusion

The *P. fluorescens* phosphate binding protein secretion leader was fused to the N-terminus of the mature domain of the human growth hormone (hGH) gene and tested for expression and secretion.

The pbp signal-sequence coding region was PCR amplified from a clone of the *P*. *fluorescens* pbp signal sequence as template, using sig_pbp for (gctctagaggaggtaacttatgaaactgaaacg) and pbp_hgh (gggaatggttgggaaggccaccgcgttggc) primers, then gel-purified. This resulted in production of an oligonucleotide fragment containing the pbp signal peptide CDS and the coding sequence for the 5' end of the mature domain of hGH.

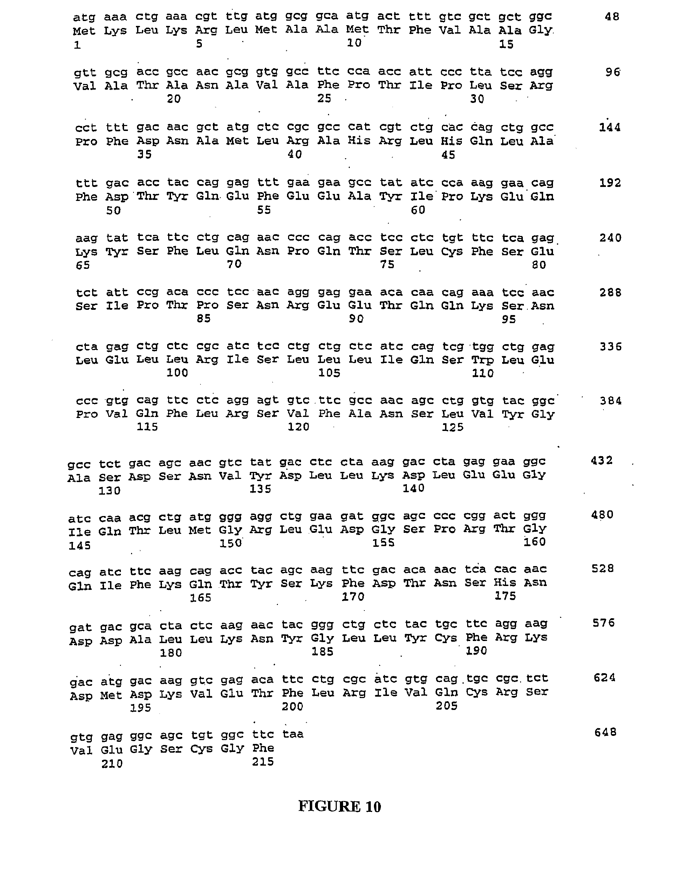

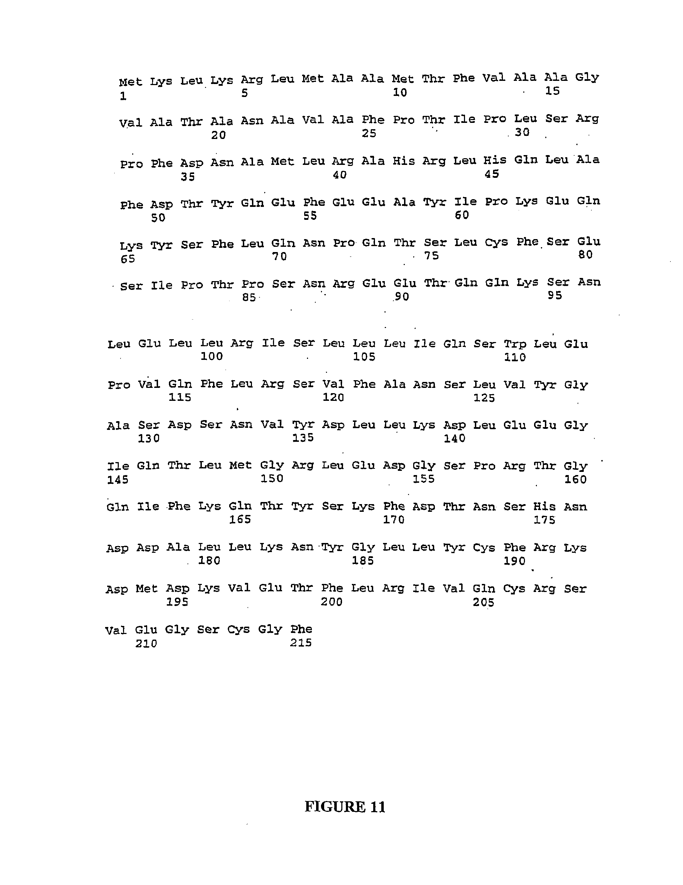

A cDNA encoding the human growth hormone was PCR-amplified from a human pituitary cDNA library (Clontech, Palo Alto CA) using primers ELVIfor (agagaactagtaaaaaggagaaatccatggctacaggctcccggacgtcc) and ELVIrev (agagactcgagtcattagaagccacagctgccctccac), which were designed to amplify only the mature domain of hGH, and cloned into pMYC1 803/SpeI XhoI, forming pDOW2400. The mature hGH gene was amplified from pDOW2400, using primers pbp_hgh_revcomp (gccaacgcggtggccttcccaaccattccc) and hgh_rev (agagactcgagtcattagaagc cacagctgccctccacagagcggcac), then purified with Strataprep columns (Stratagene) to remove primers and other reaction components. To make the polynucleotide encoding the pbp-hGH fusion, the two PCR reactions were combined and amplified again with sig_pbp for and hgh_rev in order to link the two pieces. The expected 681 bp fragment was purified with Strataprep as above, restriction digested with XbaI and XhoI and ligated to dephosphorylated pDOW1269/XhoISpeI to form pDOW 1323-10, placing pbp-hGH under control of the tac promoter in a vector analogous to pMYC1803, but with a pyrF selectable marker in place of a tetR tetracycline resistance marker gene. The ligation mix was transformed into MB101 pyrF proC lacI^{Q1}. Inserts were sequenced by The Dow Chemical Company using the method described above. The DNA and amino acid sequence of this fusion is presented in (Figure 10) and (Figure 11), respectively.

The resulting strains were tested first at the shake flask scale. Induced bands of the expected size for processed and unprocessed (22.2 kDa and 24.5 kDa, respectively) were detected by SDS-PAGE. About half of the protein was processed (indicating localization to the periplasm), and of the processed about half was in the soluble fraction and half in the insoluble fraction. Expression studies were scaled up to 20-L bioreactors. Densitometry of the Coomassie-stained SDS-PAGE gels showed that 18% of the total hGH produced was processed and soluble. The strain produced 3.2 g/L of all forms of hGH; processed and soluble was 0.6 g/L.

### Construction, Expression, and Characterization of pbp-scFv Fusion

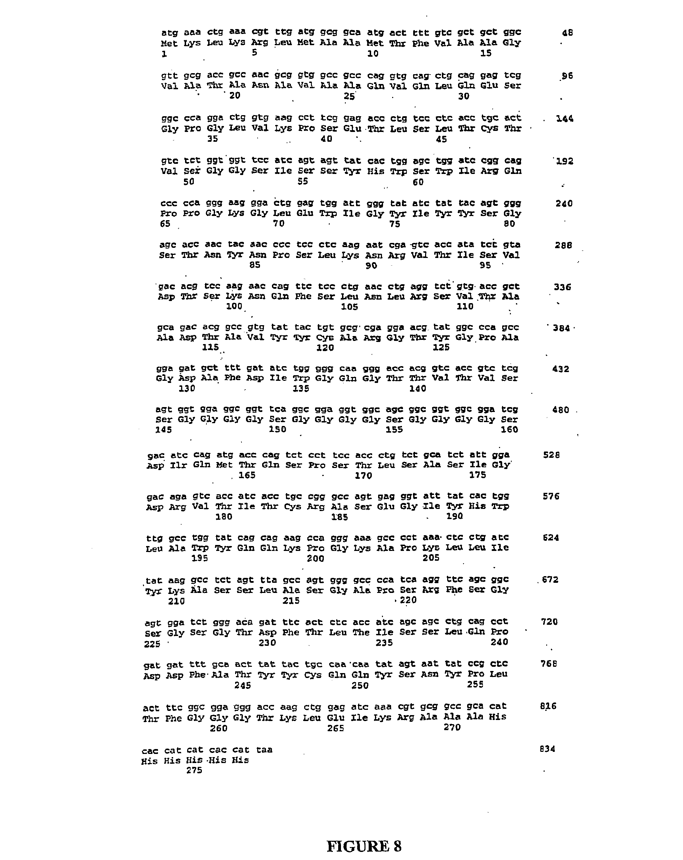

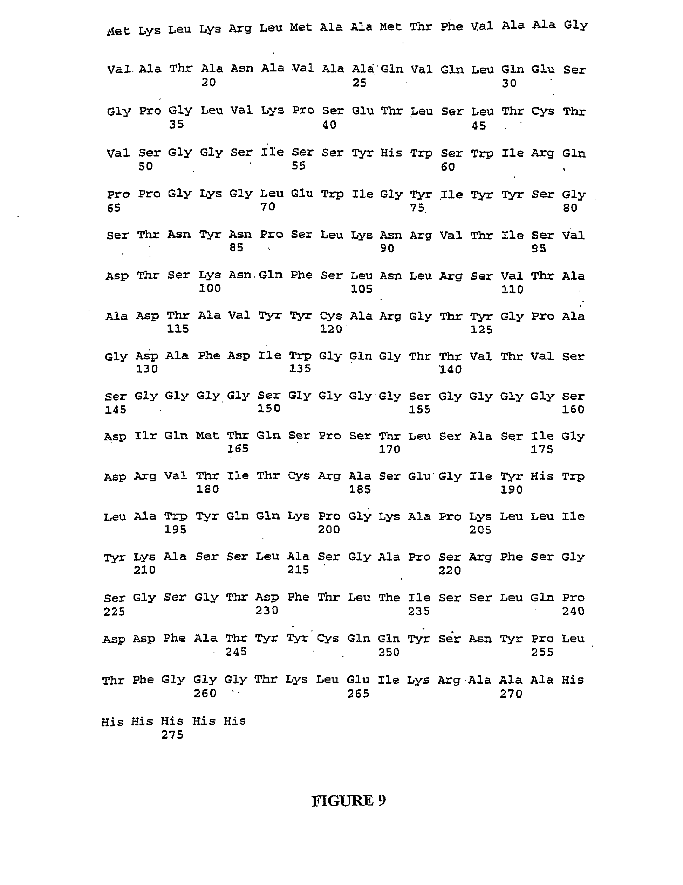

The putative 24 amino acid signal sequence of phosphate binding protein (i.e. including Metl) was fused to the open reading frame of the gal2 scFv gene (gal2) at the +2 amino acid (Ala) position. See Figure 8 and Figure 9. The signal sequence appears to be processed, indicating secretion to the periplasm. Moreover, there is secretion to the broth, in that protein was detected in the cell free culture supernatant. Surprisingly, fusion to the phosphate binding protein signal sequence appears to improve expression of gal2 scFv in *P. fluorescens.* Without the secretion signal fused at the amino terminus, expression of gal2 scFv was not detectable.

### Cloning of Gal2

PCR was performed using primers sig_pbp for (above) and pbp_gal2SOE rev (ctgcacctgggcggccaccgcgtt), which contains a reverse complement of pbp_gal2SOE for (aacgcggtggccgcceaggtgcag), and using a plasmid encoding the *P. fluorescens* pbp secretion signal peptide as template. This resulted in production of an oligonucleotide fragment containing the pbp signal peptide coding sequence (CDS) and a CDS for the 5' end of the gal2 single chain antibody (scAb or scFv).

PCR was performed using primers pbp_gal2SOE for and scFv2rev (acgcgtcgacttattaatggtg atgatggtgatgtgcggccgcacgtttgatc), and using a gal2-encoding polynucleotide as template. This resulted in production of a polynucleotide fragment containing a CDS encoding the 3'end of the pbp signal peptide and the open reading frame (ORE) encoding gal2.

Reaction products were purified. About 15ng of each was used as a "template" DNA in a further PCR reaction using primers sig_pbp_for and scFv2rev. This resulted in production of a nucleic acid fragment with the pbp signal peptide CDS fused to the gal2 coding sequence.

The predicted -I amino acid of the signal sequence (that is the last amino acid prior to the proposed cleavage site) was fused to the +2 amino acid of the gal2 scFv (Ala). The resulting fusion was cloned into the *P. fluorescens* vector pMYC1803 under control of the Ptac promoter to produce plasmid and pDOW1123 (pbp:gal2). The plasmid was transformed into *P. fluorescens* strain MB 101 carrying plasmid pCN51-lacI (described in U.S. Application No. 10/994,138, filed November 19,2004.

### Fusion of the putative phosphate binding protein signal sequence to gal2 scFv

The phosphate binding protein signal sequence was fused to a single chain antibody gene and tested for secretion to the periplasm and/or to the culture supernatant.

**Table 13: secreted Gal2fermentation summary (*compared to BSA standards)**

| | ***E. coli*** | ***P. fluorescens*** | ***Pf*/*Ec*** |
|---|---|---|---|
| **Fermentation Time (hr)** | 8-9 | 50-70 | 8 |
| **Max hGH titre (*g/L)** | 1.6 (0.8 processed) | 9.3 (25%cv) | 6(12) |
| **Dry biomass (g/L)** | 18 | (70) | 4 |
| **hGH/biomass (%w/w)** | 8.9 (4.4 processed) | 13 | 1.5 (3) |

The resulting strains were tested first at the shake flask scale. Induced bands of the expected size for unprocessed and processed gal2 (29kDa and 27kDa) were detected via SDS-PAGE in the insoluble protein fraction (data not shown). Expression studies were scaled up to 20L fermentation. Again, SDS-PAGE analysis showed that the majority of the induced protein is found in the insoluble protein fraction.

The Western analysis also indicated that some processed gal2 is present in the soluble protein fraction for pbp:gal2 (pDOW1123). Western analysis of periplasmic fractions prepared from strains carrying pDOW 1123 (using the Epicentre periplast kit) showed the presence of soluble gal2 protein.

Recombinant gal2 scFv was isolated from the cell extract of a shake flask experiment using the Qiagen Ni-NTA protocol, then refolded as described in P. Martineau et al., J Mol. Biol. 280:117-127 (1998). This antibody was found to be active against β-galactosidase in an ELISA assay.

## Claims

1. A method for producing a recombinant mammalian protein in a *Pseudomonas fluorescens* host cell comprising:
transforming a host cell with a nucleic acid encoding a recombinant mammalian protein;
growing the cell under conditions that allow expression of the recombinant mammalian protein, wherein the recombinant mammalian protein is present in the host cell in a soluble or insoluble form; and wherein the recombinant mammalian protein is operably linked to a periplasmic secretion leader coding sequence which targets the protein to the periplasm; and wherein the protein is expressed at an increased level when compared to an expression level of the protein under substantially comparable conditions in an *E. coli* expression system.

2. The method of claim 1, wherein the recombinant mammalian protein is present in the host cell in a soluble form.

3. The method of claim 1 or claim 2, further comprising isolating the recombinant mammalian protein.

4. The method of any one of claims 1 to 3, further comprising substantially purifying the recombinant mammalian protein.

5. The method of any one of claims 1 to 4, wherein the recombinant mammalian protein is present in the cell in an active form.

6. The method of any one of claims 1 to 5, wherein the recombinant mammalian protein is a human peptide.

7. The method of any one of claims 1 to 6, wherein the recombinant protein is expressed as at least 5% of the total cell protein.

8. The method of claim 1, wherein the recombinant protein is produced at a concentration of at least 10g/L.

9. The method of any one of claims 1 to 8, wherein the recombinant mammalian protein has a mass of between at least 1 kD and about 500 kD.

10. The method according to any one of claims 1 to 9, wherein the recombinant mammalian protein is an antibody or an antibody fragment.

11. The method of claim 10, wherein each sub-unit is encoded by nucleic acid on a different vector.

12. The method of claim 11 wherein each expression vector comprises one or more sequences selected from the group consisting of: one or more selection markers, an internal ribosome entry site, an activator sequence, a promoter and/or a terminator sequence.

13. The method according to claim 11 or 12, wherein an expression vector comprises a first promoter-cistron pair for expression of an immunoglobulin light chain and a second promoter-cistron pair for expression of an immunoglobin heavy chain.

14. The method according to claim 10, wherein the nucleic acid for expression of light chain and heavy chain are located on the same plasmid.

15. The method according to claim 13, wherein said first and second promoter-cistron pair each further comprises a translation initiation region operably linked to the nucleic acid encoding an antibody.

16. The method according to claim 15, wherein the translation initiation regions provide different translational strengths to the first and second promoter-cistron pair.

17. A method according to claim 1, wherein said nucleic acid encodes an antibody or antibody fragment comprising at least two separate translational units respectively encoding a light and a heavy chain.

18. A method according to claim 17, wherein the translational units are expressed in sequential fashion.

19. The method according to any one of claims 10 to 18, wherein said antibody is a single chain antibody, a full chain antibody, or an antibody fragment selected from the group consisting of Fab, Fab', F(ab')₂ F(ab')₂-leucine zipper, Fv, dsFv, and anti-CD18 antibody.

20. The method according to claim 19, wherein said Fab fragment comprises a light and a heavy chain fragment.

21. The method according to any one of claims 19 to 20, wherein said Fab comprises an antigen binding site.

22. The method according to any one of claims 10 to 21, wherein said antibody or antibody fragment is a chimeric antibody, human antibody or humanized antibody.

23. The method according to any one of claims 10 to 22, wherein said method further comprises linking a secretion signal sequence to said antibody.

24. The method according to any one of claims 10 to 19 and 22 or 23, wherein said antibody is a full chain antibody.

25. The method according to claim 19 wherein the antibody or antibody fragment is a single-chain antibody.

26. The method according to claim 25, wherein said single chain antibody is provided as a fusion protein with an effector domain.

27. A *Pseudomonas fluorescens* cell comprising a nucleic acid encoding a recombinant human peptide, wherein the recombinant human peptide is operably linked to a periplasmic secretion leader coding sequence which targets the protein to the periplasm.

28. The cell of claim 27, wherein the recombinant human peptide is expressed.

29. The method according to any one of claims 15 to 23, wherein said antibody or antibody fragment target antigen is Complement C5, CBL, CD147, IL-8, HIV gp120, VLA-4, CD11a, CD18, VEGF, CD40L, anti-Id, ICAM-1, CD2, EGFR, TGF-beta 2, TNF-alpha, E-selectin, Factor VII, Her2, F glycoprotein, CD14, ICAM-3, CD80, CD4, CD23, Beta2-integrin, alpha4beta7, CD52, HLA DR, CD22, CD64, CD2, CD3, Hepatitis B antigen, CA 125, EpCAM, gpllbllla, CD20, IL5, IL4, CD25, CD33, HLA, VNR integrin, TCR alpha beta, CMV, and IgE.

## Patentansprüche

1. Verfahren zum Erzeugen eines rekombinanten Säugetierproteins in einer Wirtszelle *Pseudomonas fluorescens,* umfassend:
Umwandeln einer Wirtszelle mit einer Nukleinsäure, die ein rekombinantes Säugetierprotein kodiert;
Züchten der Zelle unter Bedingungen, die eine Expression des rekombinanten Säugetierproteins ermöglichen, wobei das rekombinante Säugetierprotein in der Wirtszelle in einer löslichen oder unlöslichen Form vorhanden ist; und wobei das rekombinante Säugetierprotein operativ mit einer periplasmischen Sekretions-Leader-Kodiersequenz verbunden ist, die das Protein auf das Periplasma richtet; und wobei das Protein mit einem erhöhten Wert im Vergleich zu einem Expressionspegel des Proteins unter im Wesentlichen vergleichbaren Bedingungen in einem *E*. *coli*-Expressionssystem exprimiert wird.

2. Verfahren nach Anspruch 1, wobei das rekombinante Säugetierprotein in der Wirtszelle in einer löslichen Form vorhanden ist.

3. Verfahren nach Anspruch 1 oder Anspruch 2, ferner umfassend ein Isolieren des rekombinanten Säugetierproteins.

4. Verfahren nach einem der Ansprüche 1 bis 3, ferner umfassend ein im Wesentlichen Aufreinigen des rekombinanten Säugetierproteins.

5. Verfahren nach einem der Ansprüche 1 bis 4, wobei das rekombinante Säugetierprotein in der Zelle in einer aktiven Form vorhanden ist.

6. Verfahren nach einem der Ansprüche 1 bis 5, wobei das rekombinante Säugetierprotein ein menschliches Peptid ist.

7. Verfahren nach einem der Ansprüche 1 bis 6, wobei das rekombinante Protein als mindestens 5 % des gesamten Zellproteins exprimiert wird.

8. Verfahren nach Anspruch 1, wobei das rekombinante Protein mit einer Konzentration von mindestens 10 g/l erzeugt wird.

9. Verfahren nach einem der Ansprüche 1 bis 8, wobei das rekombinante Säugetierprotein eine Masse von zwischen mindestens 1 kD und ca. 500 kD hat.

10. Verfahren nach einem der Ansprüche 1 bis 9, wobei das rekombinante Säugetierprotein ein Antikörper oder ein Antikörperfragment ist.

11. Verfahren nach Anspruch 10, wobei jede Untereinheit durch Nukleinsäure an einem verschiedenen Vektor kodiert wird.

12. Verfahren nach Anspruch 11, wobei jeder Expressionsvektor eine oder mehrere Sequenzen umfasst, ausgewählt aus der Gruppe bestehend aus: einem oder mehreren Auswahl-Markern, einer internen Ribosom-Eingangsstelle, einer Aktivator-Sequenz, einer Promoter- und/oder einer Terminator-Sequenz.

13. Verfahren nach Anspruch 11 oder 12, wobei ein Expressionsvektor ein erstes Promotor-Cistron-Paar zur Expression eines Immunglobulins leichter Kette und ein zweites Promotor-Cistron-Paar zur Expression eines Immunglobulins schwerer Kette umfasst.

14. Verfahren nach Anspruch 10, wobei sich die Nukleinsäure zur Expression der leichten Kette und schweren Kette an demselben Plasmid befindet.

15. Verfahren nach Anspruch 13, wobei das erste und zweite Promotor-Cistron-Paar jeweils ferner eine Translationsinitiationsregion umfasst, die operativ mit der Nukleinsäure verbunden ist, die einen Antikörper kodiert.

16. Verfahren nach Anspruch 15, wobei die Translationsinitiationsregionen dem ersten und zweiten Promotor-Cistron-Paar verschiedene translationale Stärken bereitstellt.

17. Verfahren nach Anspruch 1, wobei die Nukleinsäure einen Antikörper oder ein Antikörperfragment kodiert, der/das mindestens zwei separate translationale Einheiten umfasst, die jeweils eine leichte und eine schwere Kette kodieren.

18. Verfahren nach Anspruch 17, wobei die translationalen Einheiten auf sequenzielle Weise exprimiert werden.

19. Verfahren nach einem der Ansprüche 10 bis 18, wobei der Antikörper ein einkettiger Antikörper, ein ganzkettiger Antikörper oder ein Antikörperfragment ist, ausgewählt aus der Gruppe bestehend aus Fab, Fab', F(ab')₂ F(ab')₂-Leucin-Zipper, Fv, dsFv und Anti-CD 1 8-Antikörper.

20. Verfahren nach Anspruch 19, wobei das Fab-Fragment ein leichte-Kette und ein schwere-Kette-Fragment umfasst.

21. Verfahren nach einem der Ansprüche 19 bis 20, wobei das Fab eine Antigen-Bindungsstelle umfasst.

22. Verfahren nach einem der Ansprüche 10 bis 21, wobei der Antikörper oder das Antikörperfragment ein chimärer Antikörper, menschlicher Antikörper oder humanisierter Antikörper ist.

23. Verfahren nach einem der Ansprüche 10 bis 22, wobei das Verfahren ferner ein Verbinden einer Sekretionssignalsequenz mit dem Antikörper umfasst.

24. Verfahren nach einem der Ansprüche 10 bis 19 und 22 oder 23, wobei der Antikörper ein ganzkettiger Antikörper ist.

25. Verfahren nach Anspruch 19, wobei der Antikörper oder das Antikörperfragment ein einkettiger Antikörper ist.

26. Verfahren nach Anspruch 25, wobei der einkettige Antikörper als ein Fusionsprotein mit einer Effektordomäne bereitgestellt ist.

27. *Pseudomonas fluorescens*-Zelle, umfassend eine Nukleinsäure, die ein rekombinantes menschliches Peptid kodiert, wobei das rekombinante menschliche Peptid operativ mit einer periplasmischen Sekretions-Leader-Kodiersequenz verbunden ist, die das Protein auf das Periplasma richtet.

28. Zelle nach Anspruch 27, wobei das rekombinante menschliche Peptid exprimiert wird.

29. Verfahren nach einem der Ansprüche 15 bis 23, wobei das Zielantigen des Antikörpers oder Antikörperfragments ein Komplement C5, CBL, CD147, IL-8, HIV gp120, VLA-4, CD11a, CD18, VEGF, CD40L, anti-id, ICAM-1, CD2, EGFR, TGF-beta 2, TNF-alpha, E-Selektin, Faktor VII, Her2, F Glykoprotein, CD14, ICAM-3, CD80, CD4, CD23, Beta2-Integrin, alpha4beta7, CD52, HLA DR, CD22, CD64, CD2, CD3, Hepatitis-B-Antigen, CA 125, EpCAM, gpIIbIIIa, CD20, IL5, IL4, CD25, CD33, HLA, VNR-Integrin, TCR alpha beta, CMV und IgE ist.

## Revendications

1. Procédé de production d'une protéine mammifère recombinante dans une cellule hôte de *Pseudomonas fluorescens* comprenant :
la transformation d'une cellule hôte avec un acide nucléique codant une protéine mammifère recombinante ;
la culture de la cellule dans des conditions qui permettent l'expression de la protéine mammifère recombinante, ladite protéine mammifère recombinante étant présente dans la cellule hôte sous forme soluble ou insoluble ; et ladite protéine mammifère recombinante étant liée fonctionnellement à une séquence codante leader de sécrétion périplasmique qui cible la protéine dans le périplasme ; et ladite protéine étant exprimée à un niveau accru lorsqu'il est comparé à un niveau d'expression de la protéine dans des conditions sensiblement comparables dans un système d'expression d'*E. coli.*

2. Procédé selon la revendication 1, ladite protéine mammifère recombinante étant présente dans la cellule hôte sous forme soluble.

3. Procédé selon la revendication 1 ou 2, comprenant en outre l'isolement de la protéine mammifère recombinante.

4. Procédé selon l'une quelconque des revendications 1 à 3, comprenant en outre la purification substantielle de la protéine mammifère recombinante.

5. Procédé selon l'une quelconque des revendications 1 à 4, ladite protéine mammifère recombinante étant présente dans la cellule sous forme active.

6. Procédé selon l'une quelconque des revendications 1 à 5, ladite protéine mammifère recombinante étant un peptide humain.

7. Procédé selon l'une quelconque des revendications 1 à 6, ladite protéine recombinante étant exprimée sous la forme d'au moins 5 % de la protéine cellulaire totale.

8. Procédé selon la revendication 1, ladite protéine recombinante étant produite à une concentration d'au moins 10 g/l.

9. Procédé selon l'une quelconque des revendications 1 à 8, ladite protéine mammifère recombinante ayant une masse comprise entre au moins 1 kD et environ 500 kD.

10. Procédé selon l'une quelconque des revendications 1 à 9, ladite protéine mammifère recombinante étant un anticorps ou un fragment d'anticorps.

11. Procédé selon la revendication 10, chaque sous-unité étant codée par un acide nucléique sur un vecteur différent.

12. Procédé selon la revendication 11, chaque vecteur d'expression comprenant une ou plusieurs séquences choisies dans le groupe constitué par : un ou plusieurs marqueurs de sélection, un site d'entrée ribosomique interne, une séquence activatrice, un promoteur et/ou une séquence terminatrice.

13. Procédé selon la revendication 11 ou 12, un vecteur d'expression comprenant une première paire promoteur-cistron pour l'expression d'une chaîne légère d'immunoglobuline et une seconde paire promoteur-cistron pour l'expression d'une chaîne lourde d'immunoglobuline.

14. Procédé selon la revendication 10, ledit acide nucléique pour l'expression de la chaîne légère et de la chaîne lourde étant situé sur le même plasmide.

15. Procédé selon la revendication 13, lesdites première et seconde paires promoteur-cistron comprenant en outre chacune une région d'initiation de traduction liée fonctionnellement à l'acide nucléique codant un anticorps.

16. Procédé selon la revendication 15, lesdites régions d'initiation de traduction fournissant des puissances de traduction différentes aux première et seconde paires promoteur-cistron.

17. Procédé selon la revendication 1, ledit acide nucléique codant un anticorps ou un fragment d'anticorps comprenant au moins deux unités de traduction distinctes codant respectivement une chaîne légère et une chaîne lourde.

18. Procédé selon la revendication 17, lesdites unités de traduction étant exprimées de façon séquentielle.

19. Procédé selon l'une quelconque des revendications 10 à 18, ledit anticorps étant un anticorps à chaîne unique, un anticorps à chaîne complète ou un fragment d'anticorps choisi dans le groupe constitué par Fab, Fab', F(ab')₂ F(ab')₂-zipper de leucine, Fv, dsFv et un anticorps anti-CD18.

20. Procédé selon la revendication 19, ledit fragment Fab comprenant un fragment de chaîne légère et un fragment de chaîne lourde.

21. Procédé selon l'une quelconque des revendications 19 à 20, ledit Fab comprenant un site de liaison d'antigène.

22. Procédé selon l'une quelconque des revendications 10 à 21, ledit anticorps ou fragment d'anticorps étant un anticorps chimérique, un anticorps humain ou un anticorps humanisé.

23. Procédé selon l'une quelconque des revendications 10 à 22, ledit procédé comprenant en outre la liaison d'une séquence signal de sécrétion audit anticorps.

24. Procédé selon l'une quelconque des revendications 10 à 19 et 22 ou 23, ledit anticorps étant un anticorps à chaîne complète.

25. Procédé selon la revendication 19, ledit anticorps ou fragment d'anticorps étant un anticorps à chaîne unique.

26. Procédé selon la revendication 25, ledit anticorps à chaîne unique étant fourni sous la forme d'une protéine de fusion possédant un domaine effecteur.

27. Cellule de *Pseudomonas fluorescens* comprenant un acide nucléique codant un peptide humain recombinant, ledit peptide humain recombinant étant lié fonctionnellement à une séquence codante leader de sécrétion périplasmique qui cible la protéine dans le périplasme.

28. Cellule selon la revendication 27, ledit peptide humain recombinant étant exprimé.

29. Procédé selon l'une quelconque des revendications 15 à 23, ledit antigène cible d'anticorps ou de fragment d'anticorps étant le complément C5, le CBL, le CD147, l'IL-8, la gp120 du VIH, le VLA-4, le CD11a, le CD18, le VEGF, le CD40L, un l'anti-Id, l'ICAM-1, le CD2, l'EGFR, le TGF-bêta 2, le TNF-alpha, la sélectine E, le facteur VII, le Her2, la glycoprotéine F, le CD14, l'ICAM-3, le CD80, le CD4, le CD23, la bêta2-intégrine, l'alpha4bêta7, le CD52, le HLA-DR, le CD22, le CD64, le CD2, le CD3, un antigène de l'hépatite B, la CA-125, l'EpCAM, la gpIIbIIIa, le CD20, l'IL5, l'IL4, le CD25, le CD33, le HLA, l'intégrine VNR, le TCR alpha bêta, le CMV et l'IgE.


### SEQUENCE LISTING

<110> Dow Global Technologies Inc.
<120> Expression of Mammalian Proteins in Pseudomonas fluorescens
<130> P114122EP
<140> 05705852.1
   <141> 2005-01-18
<150> US 60/537,148
   <151> 2004-01-16
<150> US 60/564,798
   <151> 2004-04-22
<160> 27
<170> PatentIn version 3.3
<210> 1
   <211> 609
   <212> PRT
   <213> Homo sapiens
<400> 1
<210> 2
   <211> 698
   <212> PRT
   <213> Homo sapiens
<400> 2
<210> 3
   <211> 333
   <212> DNA
   <213> Homo sapiens
<400> 3
   atggccctgt ggatgcgcct cctgcccctg ctggcgctgc tggccctctg gggacctgac 60
   ccagccgcag cctttgtgaa ccaacacctg tgcggctcac acctggtgga agctctctac 120
   ctagtgtgcg gggaacgagg cttcttctac acacccaaga cccgccggga ggcagaggac 180
   ctgcaggtgg ggcaggtgga gctgggcggg ggccctggtg caggcagcct gcagcccttg 240
   gccctggagg ggtccctgca gaagcgtggc attgtggaac aatgctgtac cagcatctgc 300
   tccctctacc agctggagaa ctactgcaac tag 333
<210> 4
   <211> 49
   <212> DNA
   <213> Artificial
<220>
   <223> hGH-sig
<400> 4
   agagaactag taaaaaggag aaatccatgt tcccaaccat tcccttatc 49
<210> 5
   <211> 60
   <212> DNA
   <213> Artificial
<220>
   <223> hGH-sigcorr
<400> 5
   agagaactag taaaaaggag aaatccatgt tcccaaccat tcccttatcc aggccttttg 60
<210> 6
   <211> 50
   <212> DNA
   <213> Artificial
<220>
   <223> ELVIfor
<400> 6
   agagaactag taaaaaggag aaatccatgg ctacaggctc ccggacgtcc 50
<210> 7
   <211> 38
   <212> DNA
   <213> Artificial
<220>
   <223> ELVIrev
<400> 7
   agagactcga gtcattagaa gccacagctg ccctccac 38
<210> 8
   <211> 20
   <212> PRT
   <213> pseudomonas fluorescens
<400> 8
   Lys Lys Ser Thr Leu Ala Val Ala Val Thr Leu Gly Ala Ile Ala Gln
   1               5                   10                  15
   Gln Ala Gly Ala
               20
<210> 9
   <211> 15
   <212> PRT
   <213> pseudomonas fluorescens
<400> 9
   Lys Leu Lys Arg Leu Met Ala Ala Met Thr Phe Val Ala Ala Gly
   1               5                   10                  15
<210> 10
   <211> 19
   <212> PRT
   <213> pseudomonas fluorescens
<400> 10
   Phe Ala Lys Leu Val Ala Val Ser Leu Leu Thr Leu Ala Ser Gly Gln
   1 5 10 15
   Leu Leu Ala
<210> 11
   <211> 15
   <212> PRT
   <213> pseudomonas fluorescens
<400> 11
   Ile Lys Arg Asn Leu Leu Val Met Gly Leu Ala Val Leu Leu Ser
   1 5 10 15
<210> 12
   <211> 22
   <212> PRT
   <213> pseudomonas fluorescens
<400> 12
   Gln Asn Tyr Lys Lys Phe Leu Leu Ala Ala Ala Val Ser Met Ala Phe
   1 5 10 15
   Ser Ala Thr Ala Met Ala
   20
<210> 13
   <211> 31
   <212> PRT
   <213> pseudomonas fluorescens
<400> 13
   Met Ile Arg Asp Asn Arg Leu Lys Thr Ser Leu Leu Arg Gly Leu Thr
   1 5 10 15
   Leu Thr Leu Leu Ser Leu Thr Leu Leu Ser Pro Ala Ala His Ser
   20 25 30
<210> 14
   <211> 30
   <212> DNA
   <213> Artificial
<220>
   <223> pbp_hgh_revcomp
<400> 14
   gccaacgcgg tggccttccc aaccattccc 30
<210> 15
   <211> 48
   <212> DNA
   <213> Artificial
<220>
   <223> hgh_rev
<400> 15
   agagactcga gtcattagaa gccacagctg ccctccacag agcggcac 48
<210> 16
   <211> 192
   <212> PRT
   <213> Artificial
<220>
   <223> hGH without signal sequence from construct
<400> 16
   Met Phe Pro Thr Ile Pro Leu Ser Arg Pro Phe Asp Asn Ala Met Leu
   1               5                   10                  15
   Arg Ala His Arg Leu His Gln Leu Ala Phe Asp Thr Tyr Gln Glu Phe
               20                  25                  30
   Glu Glu Ala Tyr Ile Pro Lys Glu Gln Lys Tyr Ser Phe Leu Gln Asn
           35                  40                  45
   Pro Gln Thr Ser Leu Cys Phe Ser Glu Ser Ile Pro Thr Pro Ser Asn
       50                  55                  60
   Arg Glu Glu Thr Gln Gln Lys Ser Asn Leu Glu Leu Leu Arg Ile Ser
   65                  70                  75                  80
   Leu Leu Leu Ile Gln Ser Trp Leu Glu Pro Val Gln Phe Leu Arg Ser
                   85                  90                  95
   Val Phe Ala Asn Ser Leu Val Tyr Gly Ala Ser Asp Ser Asn Val Tyr
               100                 105                 110
   Asp Leu Leu Lys Asp Leu Glu Glu Gly Ile Gln Thr Leu Met Gly Arg
           115                 120                 125
   Leu Glu Asp Gly Ser Pro Arg Thr Gly Gln Ile Phe Lys Gln Thr Tyr
       130                 135                 140
   Ser Lys Phe Asp Thr Asn Ser His Asn Asp Asp Ala Leu Leu Lys Asn
   145                 150                 155                 160
   Tyr Gly Leu Leu Tyr Cys Phe Arg Lys Asp Met Asp Lys Val Glu Thr
                   165                 170                 175
   Phe Leu Arg Ile Val Gln Cys Arg Ser Val Glu Gly Ser Cys Gly Phe
               180                 185                 190
<210> 17
   <211> 475
   <212> DNA
   <213> Homo sapiens
<400> 17
   agagaactag taaaaaggag aaatccatgc agggccaatt ttttagagaa atagaaaact      60
   taaaggagta ttttaatgca agtagcccag atgtagctaa gggtgggcct ctcttctcag     120
   aaattttgaa gaattggaaa gatgaaagtg acaaaaaaat tattcagagc caaattgtct     180
   ccttctactt caaactcttt gaaaacctca aagataacca ggtcattcaa aggagcatgg     240
   atatcatcaa gcaagacatg tttcagaagt tcttgaatgg cagctctgag aaactggagg    300
   acttcaaaaa gctgattcaa attccggtgg atgatctgca gatccagcgc aaagccataa    360
   atgaactcat caaagtgatg aatgacctgt caccaaaatc taacctcaga aagcggaaga    420
   gaagtcagaa tctctttcga ggccggagag catcaacgta atgactcgag tctct         475
<210> 18
   <211> 834
   <212> DNA
   <213> Artificial
<220>
   <223> phosphate-binding protein-gal2 single chain antibody fusion
<400> 18
   atgaaactga aacgtttgat ggcggcaatg acttttgtcg ctgctggcgt tgcgaccgcc     60
   aacgcggtgg ccgcccaggt gcagctgcag gagtcgggcc caggactggt gaagccttcg    120
   gagaccctgt ccctcacctg cactgtctct ggtggttcca tcagtagtta tcactggagc    180
   tggatccggc agcccccagg gaagggactg gagtggattg ggtatatcta ttacagtggg    240
   agcaccaact acaacccctc cctcaagaat cgagtcacca tatctgtaga cacgtccaag    300
   aaccagttct ccctgaacct gaggtctgtg accgctgcag acacggccgt gtattactgt    360
   gcgcgaggaa cgtatggccc agccggagat gcttttgata tctgggggca agggaccacg    420
   gtcaccgtct cgagtggtgg aggcggttca ggcggaggtg gcagcggcgg tggcggatcg    480
   gacatccaga tgacccagtc tccttccacc ctgtctgcat ctattggaga cagagtcacc    540
   atcacctgcc gggccagtga gggtatttat cactggttgg cctggtatca gcagaagcca    600
   gggaaagccc ctaaactcct gatctataag gcctctagtt tagccagtgg ggccccatca    660
   aggtccagcg gcagtggatc tgggacagat ttcactctca ccatcagcag cctgcagcct    720
   gatgattttg caacttatta ctgccaacaa tatagtaatt atccgctcac tttcggcgga    780
   gggaccaagc tggagatcaa acgtgcggcc gcacatcacc atcatcacca ttaa          834
<210> 19
   <211> 276
   <212> PRT
   <213> Artificial
<220>
   <223> phosphate-binding protein-gal2 single chain antibody fusion
<400> 19
   Met Lys Leu Lys Arg Leu Met Ala Ala Met Thr Phe Val Ala Ala Gly
   1               5                   10                  15
   Val Ala Thr Ala Asn Ala Val Ala Ala Gln Val Gln Leu Gln Glu Ser
               20                  25                  30
   Gly Pro Gly Leu Val Lys Pro Ser Glu Thr Leu Ser Leu Thr Cys Thr
           35                  40                  45
   Val Ser Gly Gly Ser Ser Ser Tyr His Trp Ser Trp Ile Arg Gln Pro
   50 55 60
   Pro Gly Lys Gly Leu Glu Trp Ile Gly Tyr Ile Tyr Tyr Ser Gly Ser
   65 70 75 80
   Thr Asn Tyr Asn Pro Ser Leu Lys Asn Arg Val Thr Ile Ser Val Asp
   85 90 95
   Thr Ser Lys Asn Gln Phe Ser Leu Asn Leu Arg Ser Val Thr Ala Ala
   100 105 110
   Asp Thr Ala Val Tyr Tyr Cys Ala Arg Gly Thr Tyr Gly Pro Ala Gly
   115 120 125
   Asp Ala Phe Asp Ile Trp Gly Gln Gly Thr Thr Val Thr Val Ser Ser
   130 135 140
   Gly Gly Gly Gly Ser Gly Gly Gly Gly Ser Gly Gly Gly Gly Ser Asp
   145 150 155 160
   Ile Gln Met Thr Gln Ser Pro Ser Thr Leu Ser Ala Ser Ile Gly Asp
   165 170 175
   Arg Val Thr Ile Thr Cys Arg Ala Ser Glu Gly Ile Tyr His Trp Leu
   180 185 190
   Ala Trp Tyr Gln Gln Lys Pro Gly Lys Ala Pro Lys Leu Leu Ile Tyr
   195 200 205
   Lys Ala Ser Ser Leu Ala Ser Gly Ala Pro Ser Arg Phe Ser Gly Ser
   210 215 220
   Gly Ser Gly Thr Asp Phe Thr Leu Thr Ile Ser Ser Leu Gln Pro Asp
   225 230 235 240
   Asp Phe Ala Thr Tyr Tyr Cys Gln Gln Tyr Ser Asn Tyr Pro Leu Thr
   245 250 255
   Phe Gly Gly Gly Thr Lys Leu Glu Ile Lys Arg Ala Ala Ala His His
   260 265 270
   His His His His
   275
<210> 20
   <211> 648
   <212> DNA
   <213> Artificial
<220>
   <223> phosphate binding protein-human growth hormone fusion
<400> 20
   atgaaactga aacgtttgat ggcggcaatg acttttgtcg ctgctggcgt tgcgaccgcc      60
   aacgcggtgg ccttcccaac cattccctta tccaggcctt ttgacaacgc tatgctccgc     120
   gcccatcgtc tgcaccagct ggcctttgac acctaccagg agtttgaaga agcctatatc     180
   ccaaaggaac agaagtattc attcctgcag aacccccaga cctccctctg tttctcagag     240
   tctattccga caccctccaa cagggaggaa acacaacaga aatccaacct agagctgctc     300
   cgcatctccc tgctgctcat ccagtcgtgg ctggagcccg tgcagttcct caggagtgtc     360
   ttcgccaaca gcctggtgta cggcgcctct gacagcaacg tctatgacct cctaaaggac     420
   ctagaggaag gcatccaaac gctgatgggg aggctggaag atggcagccc ccggactggg     480
   cagatcttca agcagaccta cagcaagttc gacacaaact cacacaacga tgacgcacta     540
   ctcaagaact acgggctgct ctactgcttc aggaaggaca tggacaaggt cgagacattc     600
   ctgcgcatcg tgcagtgccg ctctgtggag ggcagctgtg gcttctaa                  648
<210> 21
   <211> 215
   <212> PRT
   <213> Artificial
<220>
   <223> phosphate binding protein-human growth hormone fusion
<400> 21
   Met Lys Leu Lys Arg Leu Met Ala Ala Met Thr Phe Val Ala Ala Gly
   1               5                   10                  15
   Val Ala Thr Ala Asn Ala Val Ala Phe Pro Thr Ile Pro Leu Ser Arg
               20                  25                  30
   Pro Phe Asp Asn Ala Met Leu Arg Ala His Arg Leu His Gln Leu Ala
           35                  40                  45
   Phe Asp Thr Tyr Gln Glu Phe Glu Glu Ala Tyr Ile Pro Lys Glu Gln
       50                  55                  60
   Lys Tyr Ser Phe Leu Gln Asn Pro Gln Thr Ser Leu Cys Phe Ser Glu
   65                  70                  75                  80
   Ser Ile Pro Thr Pro Ser Asn Arg Glu Glu Thr Gln Gln Lys Ser Asn
                   85                  90                  95
   Leu Glu Leu Leu Arg Ile Ser Leu Leu Leu Ile Gln Ser Trp Leu Glu
               100                 105                 110
   Pro Val Gln Phe Leu Arg Ser Val Phe Ala Asn Ser Leu Val Tyr Gly
           115                 120                 125
   Ala Ser Asp Ser Asn Val Tyr Asp Leu Leu Lys Asp Leu Glu Glu Gly
       130                 135                 140
   Ile Gln Thr Leu Met Gly Arg Leu Glu Asp Gly Ser Pro Arg Thr Gly
   145                 150                 155                 160
   Gln Ile Phe Lys Gln Thr Tyr Ser Lys Phe Asp Thr Asn Ser His Asn
                   165                 170                 175
   Asp Asp Ala Leu Leu Lys Asn Tyr Gly Leu Leu Tyr Cys Phe Arg Lys
               180                 185                 190
   Asp Met Asp Lys Val Glu Thr Phe Leu Arg Ile Val Gln Cys Arg Ser
           195                 200                 205
   Val Glu Gly Ser Cys Gly Phe
       210                 215
<210> 22
   <211> 33
   <212> DNA
   <213> Artificial
<220>
   <223> sig_pbp
<400> 22
   gctctagagg aggtaactta tgaaactgaa acg 33
<210> 23
   <211> 30
   <212> DNA
   <213> Artificial
<220>
   <223> pbp_hgh
<400> 23
   gggaatggtt gggaaggcca ccgcgttggc 30
<210> 24
   <211> 24
   <212> DNA
   <213> Artificial
<220>
   <223> pbp_gal2SOE rev
<400> 24
   ctgcacctgg gcggccaccg cgtt 24
<210> 25
   <211> 25
   <212> DNA
   <213> Artificial
<220>
   <223> pbp_gal2SOE for
<400> 25
   aaccgcggtg gccgcccagg tgcag 25
<210> 26
   <211> 31
   <212> DNA
   <213> Artificial
<220>
   <223> scFv2rev
<400> 26
   atgatggtga tgtgcggccg cacgtttgat c 31
<210> 27
   <211> 22
   <212> DNA
   <213> Artificial
<220>
   <223>
<400> 27
   acgcgtcgac ttattaatgg tg 22